# FASCICULE DE BREVET EUROPEEN

(11) **EP 1 339 719 B1**
(45) Date de publication et mention de la délivrance du brevet: **16.06.2004**
(21) Numéro de dépôt: 01997491.4
(22) Date de dépôt: 21.11.2001
(51) Int. Cl.: C07D 487/06, A61K 31/5517, A61P 9/00, A61P 25/00, A61P 29/00

(54) **DERIVES DE BENZIMIDAZOLE, LEUR PREPARATION ET LEUR APPLICATION EN THERAPEUTIQUE**
BENZIMIDAZOLDERIVATE, IHRE HERSTELLUNG UND THERAPEUTISCHE VERWENDUNG
BENZIMIDAZOLE DERIVATIVES, PREPARATION AND THERAPEUTIC USE THEREOF

(30) Priorité: 23.11.2000 FR 0015141; 10.05.2001 FR 0106157
(43) Date de publication de la demande: 03.09.2003
(73) Titulaire: SANOFI-SYNTHELABO, 75013 Paris (FR)
(72) Inventeur: BARTH, Francis, F-34680 Saint-Georges d'Orques (FR); BICHON, Daniel, F-34980 Saint-Gely-du-Fesc (FR); BOLKENIUS, Frank, D-7640 Kehl (DE); VAN DORSSELAER, Viviane, F-67100 Strasbourg (FR)
(74) Mandataire: Thouret-Lemaitre, Elisabeth
(86) Numéro de dépôt international: PCT/FR2001/003667
(87) Numéro de publication internationale: WO 2002/042306

(56) Documents cités:
- EP-A- 0 646 583
- EP-A- 0 732 334
- WO-A-00/32579

## Description

La présente invention a pour objet des dérivés de benzimidazole, leur préparation et leur application en thérapeutique.

Des dérivés de benzimidazole inhibiteurs de PARP sort révélés dans WO 00/32579.

La présente invention a pour objet des composés répondant à la formule (I) 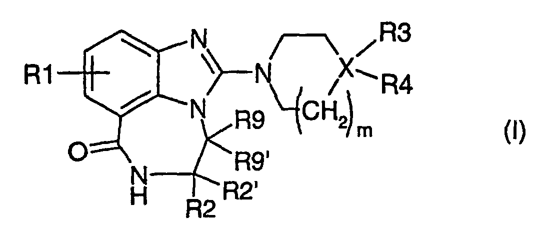 dans laquelle
**R1** représente un atome d'hydrogène, un groupe (C1-C4)alkyle, un atome d'halogène, un groupe nitro ou un groupe (C1-C4)alcoxy,
**R2, R2', R9** et **R9'** représentent indépendamment l'un de l'autre un atome d'hydrogène ou un groupe (C1-C4)alkyle,
**X** représente un atome d'azote ou un atome de carbone,
**m** est égal à 1 ou 2,
**et dans le cas où X représente un atome d'azote :**
**R3** représente un atome d'hydrogène ou un groupe (C1-C4)alkyle pour donner des composés de formule (I) comportant un ammonium quaternaire, ou encore n'existe pas pour donner des composés de formule (I) comportant une amine secondaire ou tertiaire,
R4 représente :
   un atome d'hydrogène,
   un groupe (C1-C6)alkyle,
   un groupe (C3-C7)cycloalkyle,
   un groupe 4-pipéridyle éventuellement substitué, sur un atome de carbone ou sur l'atome d'azote, par un groupe (C1-C4)alkyle,
   un groupe -(CH₂)ₚ-NR5R6, -(CH₂)ₚ-CONR5R6 ou -CO-(CH₂)ₚ-NR5R6, où p peut varier de 0 à 4 et où **R5** et **R6** représentent indépendamment l'un de l'autre un atome d'hydrogène ou un groupe (C1-C4)alkyle,
   un groupe -(CH₂)ₚ-phényle, où p peut varier de 0 à 4 (par exemple un groupe benzyle lorsque p = 1 ou un groupe phényléthyle lorsque p = 2) et où le noyau phényle est éventuellement substitué par un à trois groupes choisis indépendamment l'un de l'autre parmi : un groupe (C1-C4)alkyle, un groupe nitro, un groupe hydroxy, un groupe amino, un atome d'halogène, un groupe trifluorométhyle, un groupe (C1-C4)alcoxy, un groupe (C1-C4)alkylamino, un groupe (C1-C4)dialkylamino, un groupe -NHCHO, un groupe -NHCOR ou un groupe -NHSO₂R, où **R** représente un groupe (C1-C4)alcoxy ou un groupe (C1-C4)alkyle, ce groupe (C1-C4)alkyle pouvant être substitué par un groupe diméthylamino,
   un groupe -(CH₂)ₚ-morpholinyle, -(CH₂)ₚ-pyrrolidinyle ou -(CH₂)ₚ-tétrahydroisoquinoline, où p peut varier de 0 à 4,
   un groupe -(CH₂)ₚ-hétéroaryle, où p peut varier de 0 à 4 et où le groupe hétéroaryle est choisi parmi les groupes pyridyle, aminopyridyle, pyridinyl-N-oxyde, pyrimidinyle, pyrazinyle, pyridazinyle, imidazolyle et pyrazolyle, ledit groupe hétéroaryle pouvant être substitué, sur un atome de carbone et/ou sur un atome d'azote, par un à trois groupes choisis parmi un groupe (C1-C4)alkyle ou un groupe phényle, ce groupe phényle pouvant lui-même être substitué par un à trois groupes choisis parmi les atomes d'halogène et les groupes (C1-C4)alkyles,
   un groupe hétéroarylcarbonyle, le groupe hétéroaryle étant choisi parmi les groupes furyle, pyridyle, pyrimidyle, pyrazinyle, pyridazinyle et imidazolyle,
   un groupe phénylcarbonyle, le groupe phényle pouvant éventuellement être substitué par un atome d'halogène,
   un groupe (C1-C6)alkylcarbonyle,
   un groupe -(CH₂)ₚ-COOR' où p peut varier de 0 à 4 et où **R'** représente un atome d'hydrogène ou groupe (C1-C6)alkyle, ou bien
   un groupe phénylsulfonyle éventuellement substitué sur le noyau phényle par un atome d'halogène, un groupe trifluorométhyle, un groupe (C1-C4)alkyle, un groupe nitro ou un groupe (C1-C4)alcoxy ;
**et dans le cas où X représente un atome de carbone :**
**R3** représente un atome d'hydrogène, un groupe -NR5R6, un groupe - NHCOR7, un groupe -CONHR5, un groupe -COR7, un groupe -NHCONH₂, un groupe -OH ou un groupe -CH₂OH,
**R4** représente :
   un atome d'hydrogène,
   un groupe -(CH₂)ₚ-phényle, où p peut varier de 0 à 4 et où le noyau phényle est éventuellement substitué par un à trois groupes choisis indépendamment l'un de l'autre parmi : un groupe (C1-C4)alkyle, un groupe nitro, un groupe hydroxy, un groupe amino, un atome d'halogène, un groupe trifluorométhyle, un groupe (C1-C4)alcoxy, un groupe (C1-C4)alkylamino, un groupe (C1-C4)dialkylamino, un groupe -NHCHO, un groupe -NHCOR ou un groupe -NHSO₂R, où **R** représente un groupe (C1-C4)alcoxy ou un groupe (C1-C4)alkyle, ce groupe (C1-C4)alkyle pouvant être substitué par un groupe diméthylamino,
   un groupe -(CH₂)ₚ-hétéroaryle, où p peut varier de 0 à 4 et où le groupe hétéroaryle est choisi parmi les groupes pyridyle, aminopyridyle, pyridinyl-N-oxyde, pyrimidinyle, pyrazinyle, pyridazinyle, imidazolyle et pyrazolyle, ledit groupe hétéroaryle pouvant être substitué, sur un atome de carbone et/ou sur un atome d'azote, par un à trois groupes choisis parmi un groupe (C1-C4)alkyle ou un groupe phényle, ce groupe phényle pouvant lui-même être substitué par un à trois groupes choisis parmi les atomes d'halogène et les groupes (C1-C4)alkyles, ou bien
   un groupe -(CH₂)ₜNR7R8, où t est égal à 0 ou 1,
**R5** et **R6** sont tels que définis ci-dessus,
**R7** et **R8** représentent indépendamment l'un de l'autre un groupe (C1-C4)alkyle ou (C1-C4)alcoxy, ou forment ensemble un cycle saturé, comportant de 5 à 7 chaînons, comprenant éventuellement un atome d'azote supplémentaire, ce cycle pouvant être substitué par un groupe (C1-C4)alkyle sur un atome de carbone ou bien un atome d'azote, y compris sur l'atome d'azote sur lequel sont liés les groupes R7 et R8 pour former un ammonium quaternaire, parmi lesquels on peut citer un groupe pipéridin-1-yle, un groupe pyrrolidin-1-yle ou un groupe pipérazin-1-yle.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.
Les composés de l'invention peuvent exister à l'état de bases ou de sels d'addition à des acides pharmaceutiquement acceptables. De tels sels d'addition font également partie de l'invention.

Dans le cadre de la présente invention, on entend par :
- un groupe (Cq-Cr)alkyle : un groupe aliphatique saturé, linéaire ou ramifié, comportant de q à r atomes de carbone, q et r étant des nombres entiers. On peut notamment citer les groupes méthyle, éthyle, propyle, isopropyle, n-propyle, butyle, isobutyle, tertbutyle, n-butyle, pentyle, etc ;
- un atome d'halogène : un atome de fluor, de chlore, de brome ou d'iode,
- un groupe (C3-C7)cycloalkyle : un groupe alkyle cyclique comportant de 3 à 7 atomes de carbone. On peut notamment citer les groupes cyclopropyle, cyclobutyle, cyclopentyle, cyclohexyle et cycloheptyle.

Dans les composés de formule (I) objets de l'invention, lorsque **X** représente un atome de carbone et que **R4** représente un groupe -NR7R8, alors **R3** est de préférence différent des groupes -NR5R6, -NHCOR7, -NHCONH₂ et -OH.

Parmi les composé de formule (I) objets de la présente invention, on peut citer les composés préférés pour lesquels :
**R1, R2** et **R9** représentent indépendamment l'un de l'autre un atome d'hydrogène ou un groupe (C1-C4)alkyle,
**R2'** et **R9'** représentent des atomes d'hydrogène,
**X** représente un atome d'azote,
**m** est égal à 1 ou 2,
**R3** représente un groupe (C1-C4)alkyle pour donner des composés de formule (I) comportant un ammonium quaternaire ou encore n'existe pas pour donner des composés de formule (I) comportant une amine secondaire ou tertiaire,
**R4** représente
   un atome d'hydrogène,
   un groupe (C1-C6)alkyle,
   un groupe (C3-C7)cycloalkyle,
   un groupe 4-pipéridyle éventuellement substitué, sur un atome de carbone ou sur l'atome d'azote, par un groupe (C1-C4)alkyle,
   un groupe -(CH₂)ₚ-NR5R6, -(CH₂)ₚ-CONR5R6 ou -CO-(CH₂)ₚ-NR5R6, où p peut varier de 0 à 4 et où **R5** et **R6** représentent indépendamment l'un de l'autre un atome d'hydrogène ou un groupe (C1-C4)alkyle,
   un groupe -(CH₂)ₚ-phényle, où p peut varier de 0 à 4 et où le noyau phényle est éventuellement substitué par un à trois groupes choisis indépendamment l'un de l'autre parmi un groupe nitro, un atome d'halogène et un groupe trifluorométhyle,
   un groupe -(CH₂)ₚ-morpholinyle, -(CH₂)ₚ-pyrrolidinyle ou -(CH₂)ₚ-tétrahydroisoquinoline, où p peut varier de 0 à 4,
   un groupe -(CH₂)ₚ-pyrazolyle, où p peut varier de 0 à 4 et où le groupe pyrazolyle peut être substitué, sur un atome de carbone et/ou sur un atome d'azote, par un à trois groupes choisis parmi un groupe (C1-C4)alkyle ou un groupe phényle,
   un groupe -(CH₂)ₚ-pyridyle, où p peut varier de 0 à 4,
   un groupe hétéroarylcarbonyle, le groupe hétéroaryle étant choisi parmi un groupe furyle ou un groupe pyridyle,
   un groupe phénylcarbonyle, le groupe phényle pouvant éventuellement être substitué par un atome d'halogène,
   un groupe (C1-C6)alkylcarbonyle,
   un groupe -(CH₂)ₚ-COOR' où p peut varier de 0 à 4 et où **R'** représente un groupe (C1-C6)alkyle, ou bien
   un groupe phénylsulfonyle ;
Ou bien encore les composés préférés pour lesquels :
**R1, R2'** et **R9'** représentent des atomes d'hydrogène,
**R2** et **R9** représentent indépendamment l'un de l'autre un atome d'hydrogène ou un groupe (C1-C4)alkyle,
**X** représente un atome de carbone,
**m** est égal à 1,
**R3** représente un atome d'hydrogène, un groupe -NR5R6, un groupe - NHCOR7, un groupe -CONHR5, un groupe -COR7, un groupe -NHCONH₂, un groupe -OH ou un groupe -CH₂OH,
**R4** représente
   un atome d'hydrogène,
   un groupe phényle,
   un groupe hétéroaryle choisi parmi un groupe imidazolyle, éventuellement substitué par un groupe (C1-C4)alkyle, un groupe pyridyle, ou un groupe pyrazolyle éventuellement substitué, sur un atome d'azote ou sur un atome de carbone, par un groupe phényle pouvant lui-même être substitué par un à trois groupes choisis parmi les atomes d'halogène et les groupes (C1-C4)alkyles,
   un groupe -NR7R8,
**R5** et **R6** représentent indépendamment l'un de l'autre un atome d'hydrogène ou un groupe (C1-C4)alkyle,
**R7** et **R8** représentent indépendamment l'un de l'autre un groupe (C1-C4)alkyle ou (C1-C4)alcoxy, ou forment ensemble un cycle saturé comportant de 5 à 7 chaînons, comprenant éventuellement un atome d'azote supplémentaire, ce cycle pouvant être substitué par un groupe (C1-C4)alkyle sur un atome de carbone ou bien un atome d'azote, y compris sur l'atome d'azote sur lequel sont liés les groupes R7 et R8 pour former un ammonium quaternaire, parmi lesquels on peut citer un groupe pipéridin-1-yle, un groupe pyrrolidin-1-yle ou un groupe pipérazin-1-yle.

Parmi ces derniers composés préférés, on préfère tout particulièrement les composés de formule (I) pour lesquels :
**R1, R2** et **R9** représentent indépendamment l'un de l'autre un atome d'hydrogène ou un groupe (C1-C4)alkyle,
**R2'** et **R9'** représentent des atomes d'hydrogène,
**X** représente un atome d'azote,
**m** est égal à 1 ou 2,
**R3** représente un groupe (C1-C4)alkyle pour donner des composés de formule (I) comportant un ammonium quaternaire, ou encore n'existe pas pour donner des composés de formule (I) comportant une amine secondaire ou tertiaire,
**R4** représente
   un atome d'hydrogène,
   un groupe (C1-C4)alkyle,
   un groupe (C3-C7)cycloalkyle,
   un groupe 4-pipéridyle éventuellement substitué sur l'atome d'azote par un groupe (C1-C4)alkyle,
   un groupe -(CH₂)ₚ-NR5R6, -(CH₂)ₚ-CONR5R6 ou -CO-(CH₂)ₚ-NR5R6, où p est compris entre 1 et 3 et où **R5** et **R6** représentent indépendamment l'un de l'autre des groupes (C1-C4)alkyles,
   un groupe -(CH₂)ₚ-phényle, où p peut varier de 0 à 4 et où le noyau phényle est éventuellement substitué par un à trois groupes choisis indépendamment l'un de l'autre parmi un groupe nitro, un atome d'halogène (tel qu'un chlore ou un fluor) et un groupe trifluorométhyle,
   un groupe -(CH₂)ₚ-morpholinyle, -(CH₂)ₚ-pyrrolidinyle ou -(CH₂)ₚ-tétrahydroisoquinoline, où p est égal à 2 ou 3,
   un groupe -(CH₂)ₚ-pyrazolyle, où p peut varier de 0 à 4 et où le groupe pyrazole peut être substitué, sur un atome de carbone et/ou sur un atome d'azote, par un à trois groupes choisis parmi un groupe (C1-C4)alkyle ou un groupe phényle,
   un groupe -(CH₂)ₚ-hétéroaryle, où p est égal à 0 ou 1 et où le groupe hétéroaryle est un groupe 4-pyridyle ou 2-pyridyle,
   un groupe (C1-C4)alkylcarbonyle, ou bien
   un groupe -COOR' où **R'** représente un groupe (C1-C4)alkyle ;

Ou bien encore on préfère tout particulièrement les composés de formule (I) pour lesquels :
**R1, R2'** et **R9'** représentent des atomes d'hydrogène,
**R2** et **R9** représentent indépendamment l'un de l'autre un atome d'hydrogène ou un groupe (C1-C4)alkyle,
**X** représente un atome de carbone,
**m** est égal à 1,
**R3** représente un atome d'hydrogène, un groupe -NH₂ ou un groupe - NHCOR7,
R4 représente :
   un groupe phényle,
   un groupe hétéroaryle choisi parmi un groupe imidazolyle éventuellement substitué par un groupe (C1-C4)alkyle, ou un groupe pyrazolyle éventuellement substitué, sur un atome de carbone, par un groupe phényle pouvant lui-même être substitué par un à trois groupes choisis parmi les atomes d'halogène et les groupes (C1-C4)alkyles, ou bien
   un groupe -NR7R8, où R7 et R8 représentent indépendamment l'un de l'autre un groupe (C1-C4)alkyle ou (C1-C4)alcoxy, ou forment ensemble un cycle saturé comportant de 5 à 7 chaînons, ce cycle pouvant être substitué par un groupe (C1-C4)alkyle sur un atome de carbone ou bien un atome d'azote, y compris sur l'atome d'azote sur lequel sont liés les groupes R7 et R8 pour former un ammonium quaternaire, tel qu'un groupe pipéridin-1-yle.

D'autres composés préférés selon l'invention peuvent se définir comme suit : **R1, R2, R2', R3, X** et **m** sont tels que définis précédemment en rapport avec la formule (I), **R9** et **R9'** représentent des atomes d'hydrogène et
**dans le cas où X représente un atome d'azote, R4** représente :
un atome d'hydrogène,
un groupe (C1-C6)alkyle,
un groupe (C3-C7)cycloalkyle,
un groupe benzyle éventuellement substitué sur le noyau phényle par un atome d'halogène, un groupe trifluorométhyle, un groupe (C1-C4)alkyle, un groupe nitro ou un groupe (C1-C4)alcoxy,
un groupe phényléthyle,
un groupe hétéroaryle choisi parmi un groupe pyridyle, un groupe aminopyridyle, un groupe pyrimidinyle, un groupe pyrazinyle, un groupe pyridazinyle ou un groupe imidazolyle,
un groupe hétéroarylecarbonyle, le groupe hétéroaryle étant choisi parmi un groupe furyle, un groupe pyridyle, un groupe pyrimidyle, pyrazinyle, pyridazinyle, imidazolyle,
un groupe phénylcarbonyle, le groupe phényle pouvant éventuellement être substitué par un atome d'halogène,
un groupe (C1-C6)alkylcarbonyle,
un groupe -(CH₂)ₚCOOR où p peut varier de 0 à 4 et où **R** représente un groupe (C1-C6)alkyle,
un groupe phénylsulfonyle éventuellement substitué sur le noyau phényle par un atome d'halogène, un groupe trifluorométhyle, un groupe (C1-C4)alkyle, un groupe nitro ou un groupe (C1-C4)alcoxy, ou bien
un groupe phényle éventuellement substitué par un à trois groupes choisis indépendamment l'un de l'autre parmi : un groupe (C1-C4)alkyle, un groupe nitro, un groupe amino, un atome d'halogène, un groupe trifluorométhyle, un groupe (C1-C4)alcoxy, un groupe (C1-C4)alkylamino, un groupe (C1-C4)dialkylamino, un groupe -NHCHO ou un groupe NHCOR, où R représente un groupe (C1-C4)alcoxy
ou un groupe (C1-C4)alkyle, ce groupe (C1-C4)alkyle pouvant être substitué par un groupe diméthylamino,
**et dans le cas où X représente un atome de carbone, R4 représente :**
un atome d'hydrogène,
un groupe phényle éventuellement substitué par un à trois groupes choisis indépendamment l'un de l'autre parmi : un groupe (C1-C4)alkyle, un groupe nitro, un groupe amino, un atome d'halogène, un groupe trifluorométhyle ou un groupe (C1-C4)alcoxy,
un groupe hétéroaryle choisi parmi un groupe imidazolyle, éventuellement substitué par un groupe (C1-C4)alkyle, un groupe pyridyle, un groupe aminopyridyle, un groupe pyrimidinyle, un groupe pyrazinyle ou un groupe pyridazinyle, ou bien
un groupe -(CH₂)ₜNR7R8, où t est égal à 0 ou 1,
sous réserve que lorsque R4 représente un groupe -NR7R8, R3 est différent des groupes -NR5R6, -NHCOR7, -NHCONH₂, et -OH,
**R5** et **R6** représentent indépendamment l'un de l'autre un atome d'hydrogène ou un groupe (C1-C4)alkyle,
**R7** et **R8** représentent indépendamment l'un de l'autre un groupe (C1-C4)alkyle ou forment ensemble un cycle saturé comportant de 5 à 7 chaînons, comprenant éventuellement un atome d'azote supplémentaire, ce cycle pouvant être substitué par un groupe (C1-C4)alkyle sur un atome de carbone ou bien un atome d'azote, y compris sur l'atome d'azote sur lequel sont liés les groupes R7 et R8 pour former un ammonium quaternaire, parmi lesquels on peut citer un groupe pipéridin-1-yle, un groupe pyrrolidin-1-yle ou un groupe pipérazin-1-yle.

D'autres composés préférés de formule (I) selon l'invention peuvent encore se définir comme suit: **R1, R2, R2', R9, R9'** et **m** sont tels que définis précédemment en rapport avec la formule (I) et
**dans le cas où X représente un atome d'azote :**
**R3** est tel que défini précédemment en rapport avec la formule (I) selon l'invention, et
**R4** représente :
   un groupe 4-pipéridyle substitué, sur un atome de carbone ou sur l'atome d'azote, par un groupe (C1-C4)alkyle,
   un groupe -(CH₂)ₚ-NR5R6 ou -(CH₂)ₚ-CONR5R6, où p peut varier de 1 à 4 et où **R5** et **R6** représentent indépendamment l'un de l'autre un groupe (C1-C4)alkyle,
   un groupe -CO-(CH₂)ₚ-NR5R6, où p peut varier de 0 à 4 et où R5 et R6 représentent indépendamment l'un de l'autre un atome d'hydrogène ou un groupe (C1-C4)alkyle ;
   un groupe -(CH₂)ₚ-phényle, où p peut varier de 1 à 4 et où le noyau phényle est éventuellement substitué par un à trois groupes choisis indépendamment l'un de l'autre parmi : un groupe (C1-C4)alkyle, un groupe nitro, un groupe hydroxy, un groupe amino, un atome d'halogène, un groupe trifluorométhyle, un groupe (C1-C4)alcoxy, un groupe (C1-C4)alkylamino, un groupe (C1-C4)dialkylamino, un groupe -NHCHO ou un groupe -NHCOR, où R représente un groupe (C1-C4)alcoxy ou un groupe (C1-C4)alkyle, ce groupe (C1-C4)alkyle pouvant être substitué par un groupe diméthylamino,
   un groupe -(CH₂)ₚ-morpholinyle, -(CH₂)ₚ-pyrrolidinyle ou -(CH₂)ₚ-tétrahydroisoquinoline, où p peut varier de 1 à 4,
   un groupe -(CH₂)ₚ-hétéroaryle, où p peut varier de 1 à 4 et où le groupe hétéroaryle est choisi parmi les groupes pyridyle, aminopyridyle, pyrimidinyle, pyrazinyle, pyridazinyle, imidazolyle et pyrazolyle, ledit groupe hétéroaryle pouvant être substitué, sur un atome de carbone et/ou sur un atome d'azote, par un à trois groupes choisis parmi un groupe (C1-C4)alkyle ou un groupe phényle,
   un groupe hétéroarylcarbonyle, le groupe hétéroaryle étant choisi parmi les groupes furyle, pyridyle, pyrimidyle, pyrazinyle, pyridazinyle et imidazolyle,
   un groupe phénylcarbonyle, le groupe phényle pouvant éventuellement être substitué par un atome d'halogène,
   un groupe (C1-C6)alkylcarbonyle,
   un groupe -(CH₂)ₚ-COOR' où p peut varier de 0 à 4 et où R' représente un atome d'hydrogène ou un groupe (C1-C6)alkyle, ou bien
   un groupe phénylsulfonyle éventuellement substitué sur le noyau phényle par un atome d'halogène, un groupe trifluorométhyle, un groupe (C1-C4)alkyle, un groupe nitro ou un groupe (C1-C4)alcoxy ;
**et dans le cas où X représente un atome de carbone :**
- soit **R3** représente un groupe -NR5R6, où **R5** et **R6** représentent indépendamment l'un de l'autre un groupe (C1-C4)alkyle ; un groupe -NHCOR7, où **R7** représente un groupe (C1-C4)alcoxy ; un groupe -CONHR5, où **R5** représente un atome d'hydrogène ou un groupe (C1-C4)alkyle ; un groupe -COR7, où **R7** représente un groupe (C1-C4)alkyle ou (C1-C4)alcoxy ; ou un groupe -NHCONH₂, **R4** étant tel que défini précédemment en rapport avec la formule (I) selon l'invention,
- soit **R3** est tel que défini précédemment en rapport avec la formule (I) selon l'invention et **R4** représente :
   un groupe phényle substitué par un à trois groupes choisis indépendamment l'un de l'autre parmi : un groupe (C1-C4)alkyle ou un groupe trifluorométhyle,
   un groupe imidazolyle substitué par un groupe (C1-C4)alkyle,
   un groupe pyrazolyle substitué, sur un atome d'azote ou sur un atome de carbone, par un groupe phényle pouvant lui-même être substitué par un à trois groupes choisis parmi les atomes d'halogène et les groupes (C1-C4)alkyles,
   un groupe -CH₂-NR7R8, où **R7** et **R8** sont tels que définis dans la revendication 1, ou bien
   un groupe -NR7R8, où **R7** et **R8** représentent indépendamment l'un de l'autre un groupe (C1-C4)alkyle ou forment ensemble un cycle saturé comportant de 5 à 7 chaînons, comprenant éventuellement un atome d'azote supplémentaire, ce cycle pouvant être substitué par un groupe (C1-C4)alkyle sur un atome de carbone ou bien un atome d'azote, y compris sur l'atome d'azote sur lequel sont liés les groupes R7 et R8 pour former un ammonium quaternaire, parmi lesquels on peut citer un groupe pipéridin-1-yle, un groupe pyrrolidin-1-yle ou un groupe pipérazin-1-yle.

Les composés préférés conformes à l'invention, tels que définis ci-dessus, peuvent se présenter sous forme d'énantiomères, de diastéréoisomères ou de mélanges de ces différentes formes, y compris les mélanges racémiques, ainsi qu'à l'état de bases ou de sels d'addition à des acides pharmaceutiquement acceptables.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution par exemple. De tels groupes partant sont, par exemple, les halogènes ou un groupe hydroxy activé tel qu'un mésyle, tosyle, triflate, acétyle, etc. Des exemples de groupes partants ainsi que des références pour leur préparation sont donnés dans « Advances in Organic Chemistry », J. March, 3^{rd} Edition, Wiley Interscience, p 310-316.

Pour préparer les composés de formule (I) conformes à l'invention, on procède selon le schéma de synthèse 1 ci-dessous. Selon ce procédé, on fait réagir un dérivé de formule (II), dans laquelle R1, R2, R2', R9 et R9' sont tels que définis précédemment et A représente un groupe partant, de préférence un halogène, en présence d'une amine de formule (III), dans laquelle X, R3, R4 et m sont tels que définis précédemment, dans un solvant qui peut être un alcool, tel que l'alcool isoamylique, un éther tel que le tétrahydrofurane ou le TGME (triéthylèneglycol monométhyléther), la diméthylformamide ou bien un hydrocarbure tel que le toluène, à une température comprise entre la température ambiante et la température d'ébullition du solvant, pour obtenir le composé de formule (I). La réaction peut s'effectuer en présence d'une base telle que la 2,6-lutidine ou le tertiobutylate de sodium, en présence d'halogénures de métal alcalin tel le fluorure de potassium ou en présence de catalyseurs à base de palladium ou de nickel, comme décrit par exemple dans la demande de brevet EP 646 583 ou dans *J. Med*. *Chem*. (1986) **29** 1178-1183, *Tetrahedron Letters* (1997) **32** 5607-5610, *Tetrahedron Letters* (1999) **55** 12829-12842, *Tetrahedron Letters* (1999) **40** 6875-6879.
Lorsque le composé de formule (I) comporte une fonction amine primaire ou secondaire libre, il peut également être obtenu par réaction d'un dérivé de formule (II) avec une amine de formule (III) dans laquelle ladite fonction amine est protégée par un groupe classique de protection d'amine tel qu'un tertiobutylcarbamate (BOC). Le composé de formule (I) à fonction amine protégée ainsi obtenu est ensuite traité selon une des méthodes connues pour obtenir le composé (I) à fonction amine libre désiré. Des exemples de groupes protecteurs d'amine et de méthodes de déprotection sont notamment cités dans T.W. Greene, P.G.M. Wuts, « Protective Groups in Organic Synthesis », J. Wiley, Ed., 1991.

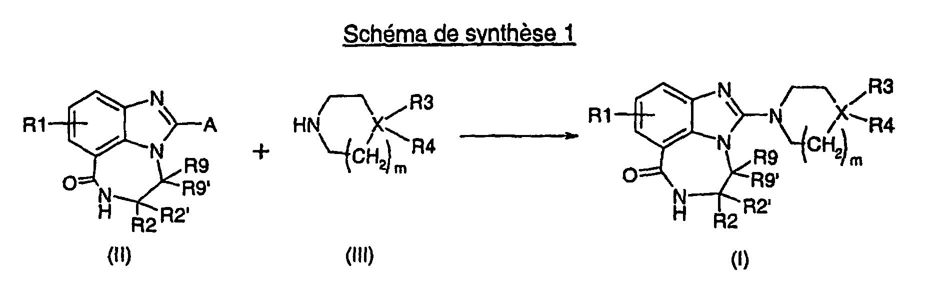

Les composés de formule (II) peuvent être préparés par une réaction de Schmidt à partir de composés de formule (X) d'après le schéma 2, selon les conditions opératoires connues de l'homme du métier, en particulier par réaction avec l'azidure de sodium dans le chloroforme en présence d'un acide tel que l'acide sulfurique. Les composés de formule (X) peuvent alternativement être préparés en une ou deux étapes en utilisant une réaction de Beckmann, soit en faisant réagir l'oxime préparée à partir de la cétone et de l'hydroxylamine avec par exemple du pentachlorure de phosphore, soit en traitant directement la cétone avec de l'acide hydroxylamine-O-sulfonique en présence d'acide formique.

Les composés de formule (X) peuvent être préparés selon des conditions opératoires connues de l'homme du métier, en particulier par réaction d'un composé de formule (IV), dans laquelle R1, R2, R2', R9 et R9' sont tels que définis précédemment, avec un agent d'halogénation tel que le chlorure de phosphoryle.

Les composés de formule (IV) peuvent être préparés selon un procédé décrit dans le schéma de synthèse 2. Selon une variante de ce procédé, une diamine de formule (V), dans laquelle R1, R2, R2', R9 et R9' sont tels que définis précédemment, est condensée avec un dérivé du phosgène tel que le carbonyle diimidazole (CDI). Selon une autre variante, un dérivé de formule (VI), dans laquelle R1 est tel que défini précédemment, est alkylé par un agent de formule (VIII, R= (C1-C4)alkyle) dans laquelle R2, R9 et R9' sont tels que définis précédemment, pour obtenir les produits de formule (VII) où R2'=H, ou par un agent de formule (IX, R= (C1-C4)alkyle et Y est un groupe partant), dans laquelle R2, R2', R9 et R9' sont tels que définis précédemment, pour obtenir les produits de formule (VII). Les composés (VII) ainsi obtenus sont ensuite transformés en acides carboxyliques (VII, R=H) ou en dérivés d'acides tel que des chlorures d'acides (VII, R=Cl), puis cyclisés selon des conditions opératoires connues de l'homme du métier pour obtenir directement les intermédiaires de formule (X). Une approche similaire à cette deuxième variante est notamment décrite dans la demande de brevet JP 55111406 ou dans *Tetrahedron Letters* (1995), **36**, 1387-1390.

Alternativement, les composés de formule (X) dans lesquels R2 et R2' ne représentent pas des atomes d'hydrogène peuvent être préparés à partir des composés (X) correspondants dans lesquels R2' représente un atome d'hydrogène par alkylation avec un réactif de type R2'Z dans lequel Z représente un groupe partant, de préférence l'iode. Cette réaction peut s'effectuer dans un solvant de type diméthylformamide, éther ou tétrahydrofurane et en présence d'une base, selon les méthodes connues de l'homme du métier.

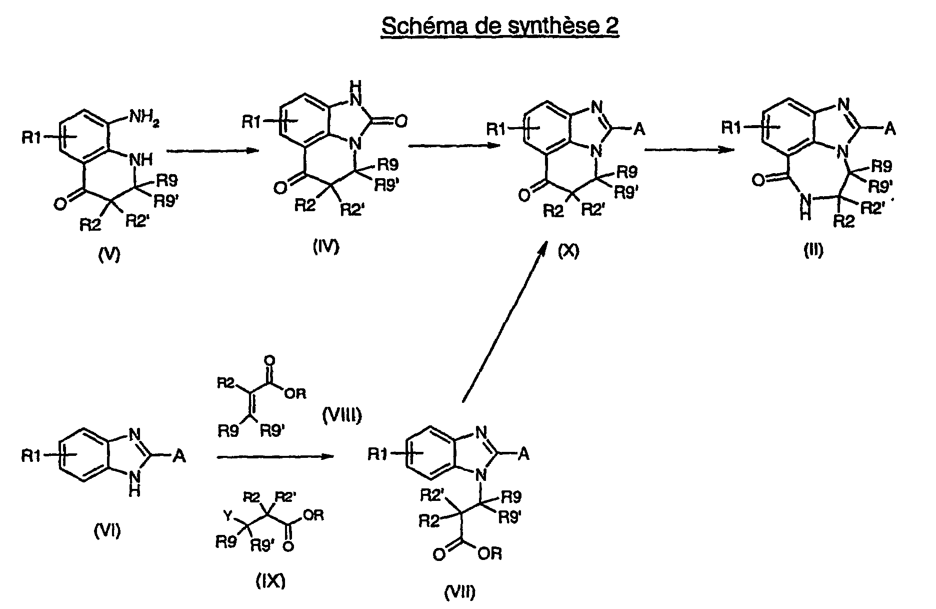

Les composés de formules (III), (V), (VI), (VIII) et (IX) sont disponibles dans le commerce ou peuvent être préparés selon des conditions opératoires connues de l'homme du métier.

La présente invention a aussi pour objet les intermédiaires de synthèse de formule (II) qui sont nouveaux.

Les exemples suivants illustrent la présente invention. Les numéros des composés exemplifiés renvoient à ceux du tableau donné plus loin, qui illustre les structures chimiques de quelques composés selon l'invention.

### Exemple 1 : Préparation d'intermédiaires de formule (II).

### 1.1. Préparation du 2-chloro-5,6-dihydro-imidazo[4,5,1-j,k][1,4] benzodiazépin-7(4H)one (A = Cl, R1 = R2 = R2' = R9 = R9' = H)

### 1.1.1. 2-chloro-4,5-dihydro-imidazo[4,5,1-i,j]quinoléin-6-one

Ce composé est obtenu à partir du composé 4*H*-imidazo[4,5,-*i,j*]-quinoléin-2,6-(1*H*,5*H*)dione (IV), décrit dans le brevet Japonais n° 55111406. Dans un tricol de 250 ml, équipé d'un reflux, on fait réagir au reflux 10 g de composé (IV) avec 38,6 ml d'oxychlorure de phosphore et 6,3 g de chlorure d'ammonium, pendant 1,5 heure. Le mélange réactionnel est ensuite refroidi, et versé sur de la glace à laquelle on ajoute une solution d'ammoniaque à 20%, sous forte agitation, jusqu'à obtention d'un pH=9. On extrait le mélange avec 2 fois 250 ml d'acétate d'éthyle, on sèche sur sulfate de magnésium, filtre et évapore. On obtient 9,81 g d'un solide blanc qu'on utilise tel quel dans l'étape suivante. RMN ¹H (200 MHz, δ ppm) DMSO D6 : 7,8 (d, 1H), 7,5 (d, 1H), 7,3 (t, 1H), 4,5 (t, 2H), 3,0 (t, 2H).

### 1.1.2. 2-chloro-5,6-dihydro-imidazo[4,5,1-j,k][1,4]benzodiazépin-7(4H)one

Dans un tricol de 1 I sous atmosphère d'argon avec agitation magnétique, thermomètre à contact et ampoule d'addition, on introduit le 2-chloro-4,5-dihydro-imidazo[4,5,1-*i,j*]quinoléin-6-one (4 g, 19,35 mmoles) en solution dans le chloroforme (48,5 ml, présence d'un léger insoluble). On refroidit le mélange à 0°C (température interne), on introduit d'abord l'acide sulfurique (20 ml) goutte à goutte sur 10 minutes puis l'azidure de sodium (4,72 g, 72,6 mmoles) sous forme solide, par portions (la réaction est très exothermique au début) et enfin on laisse revenir à température ambiante et maintient sous agitation pendant 24 heures. Le mélange réactionnel est hydrolysé avec une solution saturée de carbonate de sodium (-300 ml, pH > 7) et extrait au chloroforme (3 x 200 ml), les phases organiques sont lavées à l'eau, rassemblées et séchées sur sulfate de magnésium. Après filtration et concentration, le résidu est chromatographié avec un gradient d'éther de pétrole/acétate d'éthyle de 50/50 à 100% d'acétate d'éthyle, puis avec un gradient d'acétate d'éthyle/méthanol de 95/5 à 80/20 pour donner le produit attendu (1,81 g, 42%) et l'amide isomère (0,77 g, 18%). RMN ¹H (200 MHz, δ ppm) DMSO D6 : 3,63 (m, 2H), 4,32 (m, 2H), 7,38 (dd, 1H), 7,84 (d, 1H), 7,91 (d, 1H), 8,45 (m, 1H).

### 1.2. Préparation du 2-chloro-5,6-dihydro-5-méthyl-imidazo[4,5,1-j,k][1,4]benzodiazépin-7(4H)one (A = Cl, R1 = H, R2 = CH₃, R2' = R9 = R9' = H)

### 1.2.1. 2-chloro-5-méthyl-4,5-dihydro-imidazo[4,5,1-i,j]quinoléin-6-one

### 1.2.1.1 Ester méthylique de l'acide 1-méthyl-3-(2-chloro-benzimidazol-1-yl) propionique.

Dans un tricol de 1 1 sous atmosphère d'azote avec agitation magnétique, ampoule d'addition et réfrigérant, on introduit le 2-chloro-1*H*-benzimidazole (15,25 g, 100 mmoles) en solution dans du chloroforme (100 ml). On ajoute ensuite le Triton B (47 ml, 110 mmoles) et le méthacrylate de méthyle (107 ml, 1 mole). On maintient la réaction au reflux pendant 2 heures, laisse refroidir puis évapore le chloroforme. On reprend le résidu par de l'acétate d'éthyle, lave la phase organique 3 fois à l'eau et 1 fois avec une solution saturée de chlorure de sodium. Une flash chromatographie du brut (16,0 g) sur du gel de silice (1 kg) avec un gradient d'élution 30 à 70 % d'acétate d'éthyle dans de l'éther de pétrole permet l'obtention du produit attendu sous forme de cristaux blancs (10,1 g, 40%). RMN ¹H (300 MHz, δ ppm) DMSO D6: 1,15, (d, 3H), 3,05 (m, 1H), 3,50 (s, 3H), 4,25 (dd, 1H), 4,50 (dd, 1H), 7,25 (m, 2H), 7,60 (m, 2H)

### 1.2.1.2 Acide 1-méthyl-3-(2-chloro-benzimidazol-1-yl)propionique, sel de lithium.

Dans un ballon monocol de 0,51 avec agitation magnétique, on introduit l'ester éthylique de l'étape précédente (10,11 g, 40 mmoles) en solution dans du tétrahydrofurane (120 ml) puis ajoute une solution aqueuse d'hydroxyde de lithium (1,678 g, 40 mmoles, 60 ml d'eau distillée). On laisser réagir pendant la nuit à température ambiante, évapore le tétrahydrofurane et l'eau puis reprend le résidu dans de l'éther méthylique (2 I) et agite pendant 2 heures. On filtre le précipité blanc obtenu, le lave à l'éther éthylique puis le sèche très soigneusement sous vide de pompe à palettes sur pentoxyde de phosphore pour obtenir le composé attendu sous forme de cristaux blancs (9,48 g, 97%). Le composé est utilisé tel quel pour l'étape suivante. RMN ¹H (300 MHz, δ ppm) DMSO D6 + ε D₂O: 1,35 (d, 3H), 2,60 (m, 1H), 4,10 (dd, 1H), 4,40 (dd, 1H), 7,25 (m, 2H), 7,55 (d, 1H), 7,60 (d, 1H) LC-MS: MH⁺ = 239 (acide).

### 1.2.1.3 2-chloro-5-méthyl-4,5-dihydro-imidazo[4,5,1-i,j]quinoléin-6-one.

Dans un tricol de 1 I sous atmosphère d'argon, avec agitation mécanique, réfrigérant et ampoule d'addition on introduit le sel de lithium décrit ci-dessus (4,74 g, 19,38 mmoles) puis ajoute du 1,2-dichloroéthane fraîchement distillé sur pentoxyde de phosphore (0,5 I). On ajoute rapidement sous agitation le chlorure d'oxalyle (3,40 ml, 38,75mmoles) et chauffe le mélange réactionnel pendant 15 minutes vers 40°C. On ajoute au chlorure d'acide intermédiaire ainsi obtenu, le chlorure d'aluminium (7,75 g, 58 mmoles) et chauffe le mélange au reflux pendant 3 heures. On laisse refroidir puis verse sur un mélange glace/sel et extrait au 1,2-dichloroéthane, lave la phase organique avec une solution saturée en chlorure de sodium, sèche sur sulfate de magnésium, filtre puis évapore le solvant. Une flash chromatographie du brut (4,84 g) sur gel de silice (420 g) avec élution par un gradient de 100% de dichlorométhane à un mélange 90/10: dichlorométhane/acétate d'éthyle permet l'obtention du produit attendu sous forme de cristaux blancs (3,36 g, 78%).
RMN ¹H (300 MHz, δ ppm) DMSO D6: 1,40 (d, 3H), 3,20 (m, 1H), 4,10 (dd, 1H), 4,65 (dd, 1H), 7,45 (t, 1H), 7,70 (d, 1H), 7,85 (d, 1H). LC-MS: MH⁺ = 221.

### 1.2.2. 2-chloro-5,6-dihydro-5-méthyl-imidazo[4,5,1-j,k][1,4]benzodiazépin-7(4H)one.

On opère comme dans l'exemple 1.1.2, en faisant réagir 2,6 g de dérivé chloré de formule (X), 30 ml de chloroforme, 2,85 g d'azidure de sodium et 12 ml d'acide sulfurique. On obtient après 2 chromatographies sur gel de silice le produit attendu (1,87 g, 68%) et des traces de l'amide isomère (0,08 g, 3%). RMN ¹H (300 MHz, δ ppm) CDCl₃: 1,45 (d, 3H), 4,00 (m, 1H), 4,11 (m, 1H), 4,42 (d, 1H), 7,37 (dd, 1H), 7,83 (d, 1H), 8,05 (d, 1H).

### 1.3. Préparation du 2-chloro-5,6-dihydro-10-méthyl-imidazo[4,5,1-j,k][1,4]benzodiazépin-7(4H)one (A = Cl, R1 = 10-CH₃, R2 = R'2 = R9 = R9' = H)

### 1.3.1.2-chloro-4,5-dihydro-9-méthyl-imidazo[4,5,1-i,j]quinoléin-6-one.

### 1.3.1.1. 2-hydroxy-4-méthyl-benzimidazole.

Dans un bicol de 250 ml sous atmosphère d'argon avec agitation magnétique, on introduit successivement le 2,3-diaminotoluène (5 g, 41 mmoles), le 1,1-carbonyldiimidazole (7,3 g, 45 mmoles) et 50 ml de DMF anhydre. Après chauffage du mélange à 90-95°C pendant 4h, le solvant est distillé sous vide et le résidu obtenu est repris par de l'eau (250 ml) et extrait à l'acétate d'éthyle (3 x 250 ml). L'insoluble formé lors de l'extraction est récupéré (4,8 g) et les phases organiques réunies sont relavées avec une solution saturée en NaCl, séchées sur MgSO₄, filtrées et concentrées, permettant de récupérer 1,2 g de produit désiré supplémentaire (6 g, quantitatif). RMN ¹H (300 MHz, δ ppm) DMSO D6: 2,27 (s, 3H), 6,72 (m, 2H), 6,82 (m, 1H).

### 1.3.1.2. 2-chloro-4-méthyl-benzimidazole.

Dans un bicol de 250 ml sous atmosphère d'argon avec agitation magnétique, on introduit le 2-hydroxy-4-méthyl-benzimidazole (5,92 g, 40 mmoles) et 40 ml de chlorure de phosphoryle. Le mélange est chauffé sous reflux pendant 20 heures et le chlorure de phosphoryle est évaporé sous vide. On reprend le solide obtenu dans de l'eau (250 ml), on neutralise jusqu'à pH=8 avec de l'ammoniaque à 28% et on extrait la phase aqueuse avec de l'acétate d'éthyle (3x250 ml). Après traitement usuel on obtient le 2-chloro 4-méthyl benzimidazole avec un rendement de 93% (6,23 g). RMN ¹H (300 MHz, δ ppm) DMSO D6: 2,47 (s, 3H), 7,01 (d, 1H), 7,11 (t, 1H), 7,32 (d, 1H).

### 1.3.1.3. Ester méthylique de l'acide 1-méthyl-3-(2-chloro-4-méthyl-benzimidazol-1-yl)propionique.

Ce composé est préparé selon le mode opératoire décrit dans l'exemple 1.2.1.1. (7,85 g, 94%). RMN ¹H (300 MHz, δ ppm) DMSO D6: 2,48 (s, 3H), 2,86 (t, 2H), 3,56 (s, 3H), 4,49 (t, 2H), 7,06 (d, 1H), 7,19 (t, 1H), 7,44 (d, 1H).

### 1.3.1.4. Acide 1-méthyl-3-(2-chloro-4-méthyl-benzimidazol-1-yl)propionique, sel de lithium.

Ce composé est préparé selon le mode opératoire décrit dans l'exemple 1.2.1.2 (6,94 g, 94%). RMN ¹H (300 MHz, δ ppm) DMSO D6: 2,3 (dd, 2H), 2,48 (s, 3H), 4,32 (dd, 2H), 7,01 (d, 1H), 7,15 (t, 1H), 7,41 (d, 1H) (acide).

### 1.3.1.5. 2-chloro-4,5-dihydro-9-méthyl-imidazo[4,5,1-i,j]quinoléin-6-one.

Ce composé est préparé selon le mode opératoire décrit dans l'exemple 1.2.1.3 (4,72 g, 76%). RMN ¹H (300 MHz, δ ppm) DMSO D6: 2,58 (s, 3H), 3,05 (t, 2H), 4,53 (t, 2H), 7,19, d, 1H), 7,52 (d, 1H).

### 1.3.2. 2-chloro-5,6-dihydro-10-méthyl-imidazo[4,5,1-j,k][1,4]benzodiazépin-7(4H)one.

On opère comme dans l'exemple 1.1.2, en faisant réagir 1,9 g de dérivé chloré décrit dans l'exemple 1.3.1.5, 25 ml de chloroforme, 2,10 g d'azidure de sodium et 9 ml d'acide sulfurique. On obtient après 2 chromatographies sur gel de silice (dichlorométhane/méthanol/ammoniaque: 95/5/0,5 puis un gradient de méthanol/ ammoniaque: 90/10 de 0 à 7% dans le dichlorométhane) le produit attendu (0,78 g, 38,4%) et un mélange du composé attendu et de l'amide isomère (0,77 g, 38%). RMN ¹H (300 MHz, δ ppm) DMSO D6 : 2,56 (s, 3H), 3,61 (m, 2H), 4,32 (s large, 2H), 7,19 (d, 2H), 7,79 (d, 2H), 8,35 (t, NH amide).

### Exemple 2 : 2-(4-phényl-pipérazin-1-yl)-5,6-dihydro-imidazo[4,5,1-j,k][1,4]benzodiazépin-7(4H)one (composé n° 1)

(R1 = R2 = R2' = R9 = R9' = H, R3 = -, X = N, R4 = phényle, m = 1).
On introduit successivement dans un ballon muni d'un réfrigérant avec agitation magnétique, l'intermédiaire de formule (II) du point 1.1 (0,2 g, 0,903 mmole), 1 ml de triéthylèneglycol monométhyl éther (TGME), la 2,6-lutidine (0,116 ml, 0,993 mmole), le fluorure de césium (0,137 g, 0,903 mmole) et la 1-phénylpipérazine (0,152 ml, 0,993 mmole) en solution dans du TGME (1 ml). On chauffe le mélange réactionnel à 140°C pendant 3 heures, laisse refroidir, et extrait 2 fois à l'acétate d'éthyle. On lave les phases organiques 2 fois avec de l'eau, sèche sur sulfate de sodium, filtre et concentre. On chromatographie le résidu obtenu sur gel de silice (dichlorométhane/méthanol: 98/2) et obtient le composé attendu (0,167g, 53%). LC-MS: MH+ = 348. RMN ¹H (360 MHz, δ ppm) DMSO D6: 3,32 (m, 4H), 3,45 (m, 4H), 3,51 (m, 2H), 4,21 (m, 2H), 6,82 (t, 1H), 7,02 (d, 2H), 7,20 (dd, 1H), 7,25 (m, 2H), 7,62 (d, 2H), 8,38 (m, 1H).

### Exemple 3: 2-[4-(4-pyridyl)-pipérazin-1-yl]-5,6-dihydro-imidazo[4,5,1-j,k][1,4]benzodiazépin-7(4H)one (composé n° 2)

(R1 = R2 = R2' = R9 = R9' = H, R3 = -, X = N, R4 = 4-pyridyle, m = 1)
On introduit successivement dans un ballon muni d'un réfrigérant avec agitation magnétique, l'intermédiaire de formule (II) décrit au point 1.1 (0,2 g, 0,907 mmole), du TGME (1 ml), la 2,6-lutidine (0,117 ml, 0,998 mmole), le fluorure de césium (0,138 g, 0,907 mmole) et la 1-(4-pyridyl)pipérazine (0,163 g, 0,998 mmole) en solution dans 1 ml de TGME. On chauffe le mélange réactionnel à 120°C pendant 3 heures, laisse refroidir, rajoute une solution saturée de carbonate de sodium et extrait 4 fois au dichlorométhane. On lave les phases organiques 2 fois avec une solution saturée en chlorure de sodium, sèche sur sulfate de sodium, filtre et concentre. On chromatographie le résidu obtenu sur gel de silice (dichlorométhane/méthanol/NH4OH: 95/5/0,1) pour obtenir le composé attendu sous forme d'un solide presque blanc (0,128 g, 41%). LC-MS: MH+ = 348. RMN ¹H (500 MHz, δ ppm) DMSO D6: 3,40 (m, 4H), 3,49 (m, 6H), 4,21 (t, 2H), 6,89 (d, 2H), 7,18 (dd, 1H), 761 (m, 2H), 8,2 (d, 2H), 8,35 (t, 1H)

### Exemple 4: 2-[4-(1-pipéridyl)-pipéridin-1-yl]-5,6-dihydro-imidazo[4,5,1-j,k][1,4]benzodiazépin-7(4H)one (composé n° 3)

(R1 = R2 = R2' = R9 = R9' = R3 = H, X = C, R4 = 1-pipéridyle, m = 1)
On introduit successivement dans un ballon muni d'un réfrigérant avec agitation magnétique, l'intermédiaire de formule (II) décrit au point 1.1 (0,2 g, 0,907 mmole), la TGME (1 ml), la 2,6-lutidine (0,125 ml, 1,066 mmole), le fluorure de césium (0,148 g, 0,969 mmole) et la 4-pipéridinopipéridine (0,180 g, 1,066 mmole) en solution dans 1 ml de TGME. On chauffe le mélange réactionnel à 140°C pendant 4 heures, laisse refroidir, rajoute une solution saturée de carbonate de sodium et extrait 2 fois à l'acétate d'éthyle et 4 fois au dichlorométhane. On lave les phases organiques à l'eau, sèche sur sulfate de sodium, filtre et concentre. On chromatographie le résidu sur gel de silice (dichlorométhane/méthanol/NH₄OH: 85/15/0,1) et cristallise le composé obtenu dans un mélange chloroforme/éther pour obtenir le composé du titre sous forme d'un solide blanc (0,155 g, 49%). LC-MS: MH+ = 353. RMN ¹H (500 MHz, δ ppm) DMSO D6: 1,38 (m, 2H), 1,48 (m, 4H), 1,65 (m, 2H), 1,79 (m, 2H), 2,49 (m, 1H), 2,88 (m, 2H), 3,28 (m, 4H), 3,47 (t, 2H), 3,64 (m, 2H), 4,12 (m, 2H), 7,15 (dd, 1H), 7,56 (m, 2H), 8,31 (t, 1H).

### Exemple 5 : 2-[4-(5-méthyl-1H-imidazo-4-yl)-pipéridin-1-yl]-5,6-dihydro-imidazo[4,5,1-j,k][1,4]benzodiazépin-7(4H)one (composé n° 4)

(R1 = R2 = R2' = R3 = R9 = R9' = H, X = C, R4 = 5-méthyl-1*H*-imidazo-4-yle, m = 1)
On introduit successivement dans un ballon muni d'un réfrigérant avec agitation magnétique, l'intermédiaire de formule (II) décrit au point 1.1 (0,2 g, 0,907 mmole), la DMF (1,5 ml), la 2,6-lutidine (0,117 ml, 0,998 mmole), le fluorure de césium (0,138 g, 0,907 mmole) et la 4-(5-méthyl-1*H*-imidazo-4-yl)-pipéridine (0,375 g, 2,27 mmoles) en solution dans 1 ml de DMF. On chauffe le mélange réactionnel à 140°C pendant 2 heures, laisse refroidir, rajoute une solution saturée de bicarbonate de sodium, une solution saturée de chlorure de sodium et extrait 4 fois au dichlorométhane. On lave les phases organiques 2 fois avec une solution saturée de chlorure de sodium, sèche sur sulfate de sodium; filtre et concentre. On chromatographie le résidu sur gel de silice (dichlorométhane/méthanol/NH₄OH: 90/10/0,1) et cristallise le composé obtenu dans un mélange méthanol (minimum)/dichlorométhane/éther pour obtenir le composé attendu sous forme d'un solide blanc (0,175 g, 55%). LC-MS: MH+ = 350. RMN ¹H (500 MHz, δ ppm) DMSO D6: 1,71 (m, 2H), 1,94 (m, 2H), 2,14 (m, 3H), 2,79 (m, 1H), 3,01 (m, 2H), 3,50 (m, 2H), 3,69 (m, 2H), 4,15 (m, 2H), 7,16 (dd, 1H), 7,34 (s, 1H), 7,58 (d, 2H), 8,33 (m, 1H), 11,54 (m, 1H)

### Exemple 6: 2-(4-phényl-pipérazin-1-yl)-5,6-dihydro-5-méthyl-imidazo [4,5,1-j,k][1,4]benzodiazépin-7(4H)one (composé n° 5)

(R1 = R2' = R9 = R9' = H, R3 = -, R2 = méthyle, X = N, R4 = phényle, m = 1)
On introduit successivement dans un ballon muni d'un réfrigérant avec agitation magnétique, l'intermédiaire de formule (II) du point 1.2 (0,2g, 0,849 mmole), du TGME (1 ml), la 2,6-lutidine (0,109 ml, 0,934 mmole), le fluorure de césium (0,129 g, 0,849 mmole) et la 1-phénylpipérazine (0,152 g, 0.934 mmole) en solution dans du TGME (1 ml). On chauffe le mélange réactionnel à 140°C pendant 3 heures, laisse refroidir, et extrait 2 fois à l'acétate d'éthyle. On lave les phases organiques 2 fois avec de l'eau, sèche sur sulfate de sodium, filtre et concentre. On chromatographie le résidu obtenu sur gel de silice avec l'acétate d'éthyle comme éluant. Le composé attendu est obtenu par cristallisation dans un mélange dichlorométhane/acétate d'éthyle (0,2 g, 65%). LC-MS: MH+ = 362.
RMN ¹H (500 MHz, δ ppm) DMSO D6: 1,27 (d, 3H), 3,27 (m, 2H), 3,36 (m, 4H), 3,50 (m, 2H), 3,79 (m, 1H), 4,10 (m, 2H), 6,81 (t, 1H), 7,00 (d, 2H), 7,18 (dd, 1H), 7,24 (t, 2H), 7,60 (d, 2H), 8,18 (d 1H)

### Exemple 7 : 2-(4-N-tert-butyloxycarbonyl-pipérazin-1-yl)-5,6-dihydro-imidazo[4,5,1-j,k][1,4]benzodiazépin-7(4H)one (composé n° 15)

(R1 = R2 = R2' = R9 = R9' = H, R3 = -, X = N, R4 = *tert*-butyloxycarbonyle, m = 1)
On introduit successivement dans un ballon muni d'un réfrigérant avec agitation magnétique, l'intermédiaire chloré décrit au point 1.1.2. (0,4 g, 1,9 mmoles), du TGME (4 ml), la lutidine (0,245 ml, 2,09 mmoles), le fluorure de césium (0,29 g, 1,9 mmoles) et la 4-N-*tert*-butyloxycarbonyl-pipérazine (0,369 g, 2,09 mmoles). On chauffe le mélange réactionnel à 140°C pendant 6 heures, laisse refroidir, rajoute de l'eau et extrait 2 fois au dichlorométhane. On lave les phases organiques 2 fois avec de l'eau, sèche sur sulfate de sodium, filtre et concentre. Une chromatographie du résidu sur gel de silice (gradient de 0% à 10% de méthanol dans le dichlorométhane) fournit le produit du titre (0,369 g, 56%). RMN ¹H (500 MHz, δ ppm) DMSO D6 : 1,42 (s, 9H), 3,22 (m, 4H), 3,49 (m, 4H), 4,14 (m, 2H), 7,17 (t, 1H), 7,60 (m, 2H), 8,34 (m, 1H).

### Exemple 8 : 2-(4-pipérazin-1-yl)-5,6-dihydro-imidazo[4,5,1-j,k][1,4] benzodiazépin-7(4H)one, dichlorhydrate (composé n° 16)

(R1 = R2 = R2' = R4 = R9 = R9' = H, R3 = -, X = N, m = 1)
On ajoute une solution d'acide chlorhydrique (gaz) dans de l'éther (10 ml) à une solution du composé de l'exemple 7 (0,205 g, 0,55 mmoles) dans du méthanol (20 ml) et garde le mélange sous agitation magnétique à température ambiante pendant 5 heures On concentre et le composé attendu est obtenu après cristallisation dans du méthanol (0,182 g, 96%). RMN ¹H (500 MHz, δ ppm) DMSO D6 : 3,29 (m, 2H), 3,52 (m, 4H), 3,69 (m, 4H), 4,20 (m, 2H), 7,35 (dd, 1H), 7,72 (m, 2H), 8,54 (m, 1H), 9,44 (m, 2H).

### Exemple 9 : 2-(4-N-tert-butyloxycarbony)-pipérazin-1-yl)-5-méthyl-5,6-dihydro-imidazo[4,5,1-j,k][1,4]benzodiazépin-7(4H)one (composé n° 7)

(R2 = CH₃, R1 = R2' = R9 = R9' = H, R3 = -, X = N, R4 = *tert*-butyloxycarbonyle, m = 1)
On procède de la manière indiquée dans l'exemple 7, en utilisant l'intermédiaire chloré décrit au point 1.2.2, pour obtenir le composé du titre avec un rendement de 80% (1,07 g). RMN ¹H (360 MHz, δ ppm) DMSO D6 : 1,26 (m, 3H), 1,43 (s, 9H), 3,17 (m, 2H), 3,45 (m, 2H), 3,55 (m, 2H), 3,78 (m, 2H), 4,05 (m, 2H), 7,18 (t, 1H), 7,60 (d, 2H), 8,22 (m, 1H).

### Exemple 10: 2-(4-pipérazin-1-yl)-5-méthyl-5,6-dihydro-imidazo-[4,5,1-j,k][1,4]benzodiazépin-7(4H)one, dichlorhydrate (composé n° 9)

(R2 = CH₃, R1 = R2' = R4 = R9 = R9' = H, R3 = -, X = N, m = 1)
On procède de la manière indiquée dans l'exemple 8 pour obtenir le composé du titre avec un rendement quantitatif (0,36 g). RMN ¹H (360 MHz, δ ppm) DMSO D6: 1,29 (m, 3H), 3,25 (m, 2H), 3,37 (m, 2H), 3,67 (m, 2H), 3,80 (m, 4H), 4,12 (m, 2H), 7,39 (t, 1H), 7,72 (m, 2H), 8,43 (m, 1H), 9,53 (m, 2H).

### Exemple 11 : 2-(4-phényl-4-tert-butyloxycarbonylamino-pipéridin-1-yl)-5,6-dihydro-imidazo[4,5,1-j,k][1,4]benzodiazépin-7(4H)one (composé n° 17)

(R1 = R2 = R2' = R9 = R9' = H, R4 = phényle, X = C, R3 = -NH-*tert*-butyloxycarbonyle, m = 1)
On opère comme dans l'exemple 7 en faisant réagir 0,5 g (2,26 mmoles) de dérivé chloré de l'exemple 1.1.2., 0.345 g (2,26 mmoles) de fluorure de césium, 0,29 ml (2,49 mmoles) de lutidine et 0,69 g (2,49 mmoles) de 4-phényl-4-*tert*-butyloxycarbonylamino-pipéridine dans 2 ml de TGME à 140°C pendant 3,5 heures. Après traitement usuel, purification par chromatographie sur gel de silice (gradient de méthanol dans le dichlorométhane de 0 à 12%) et cristallisation (dichlorométhane avec un peu de méthanol / éther), le composé du titre est obtenu avec un rendement de 70% (0,73 g). RMN ¹H (500 MHz, δ ppm) DMSO D6 : 1,35 (s, 9H), 2,05 (m, 2H), 2,40 (m, 2H), 3,23 (m, 2H), 3,47 (m, 4H), 4,17 (m, 2H), 7,16 (dd, 1H), 7,21 (m, 1H), 7,33 (m, 2H), 7,39 (m, 2H), 7,58 (m, 2H), 8,32 (m, 1H).

### Exemple 12: 2-(4-phényl-4-amino-pipéridin-1-yl)-5,6-dihydro-imidazo [4,5,1-j,k][1,4]benzodiazépin-7(4H)one, dichlorhydrate (composé n° 20)

(R1 = R2 = R2' = R9 = R9' = H, R4 = phényle, X = C, R3 = NH₂, m = 1)
On procède de la manière indiquée dans l'exemple 8 pour obtenir le composé du titre avec un rendement de 80% (0,403 g). RMN ¹H (360 MHz, δ ppm) DMSO D6 : 2,37 (m, 2H), 2,57 (m, 2H), 3,92 (m, 2H), 4,20 (m, 2H), 7,39 (t, 1H), 7,44 (m, 1H), 7,51 (m, 2H), 7,70 (m, 3H), 7,74 (d, 1H), 8,60 (m, 1H), 8,81 (m, 4H).

### Exemple 13: 2-(4-cyclohexyl-pipérazin-1-yl)-5,6-dihydro-10-méthyl-imidazo[4,5,1-j,k][1,4]benzodiazépin-7(4H)one (composé n° 33)

(R1 = 10-CH₃, R2 = R2' = R9 = R9' = H, R3 = -, X = N, R4 = cyclohexyle, m= 1)
On introduit successivement dans un ballon muni d'un réfrigérant avec agitation magnétique, l'intermédiaire chloré décrit au point 1.3.2. (0,2 g, 0,85 mmoles), du TGME (2 ml), la lutidine (0,109 ml, 0,935 mmoles), le fluorure de césium (0,129 g, 0,85 mmoles) et la 4-N-cyclohexyl-pipérazine (0,158 g, 0,935 mmoles). On chauffe le mélange réactionnel à 140°C pendant 8 heures. Après traitement usuel, purification par chromatographie sur gel de silice (gradient de méthanol dans le dichlorométhane de 0 à 13%) et cristallisation (dichlorométhane /éther avec un peu de pentane), le composé du titre est obtenu avec un rendement de 30% (0,092 g). RMN ¹H (500 MHz, δ ppm) DMSO D6 : 1,09 (m, 1H), 1,22 (m, 4H), 1,58 (m, 1H), 1,76 (m, 4H), 2,29 (m, 1H), 2,48 (s, 3H), 2,64 (m, 4H), 3,22 (m, 4H), 3,45 (m, 2H), 4,10 (m, 2H), 6,98 (d, 1H), 7,49 (d, 1H), 8,22 (m, 1H).

Dans le tableau qui suit :
. dans la colonne « sel », « HCl » correspond à un chlorhydrate et le rapport entre parenthèse est le rapport (acide:base),
. « Pr » représente un groupe propyle,
. les analyses RMN correspondent à des RMN du proton. Sauf mention contraire, les mesures sont effectuées dans du DMSO D6. * et ** signifient que les mesures sont effectuées, respectivement, à 360 MHz et à 500 MHz. Si aucune indication de ce genre n'est reportée, alors la mesure est effectuée à 200 MHz.

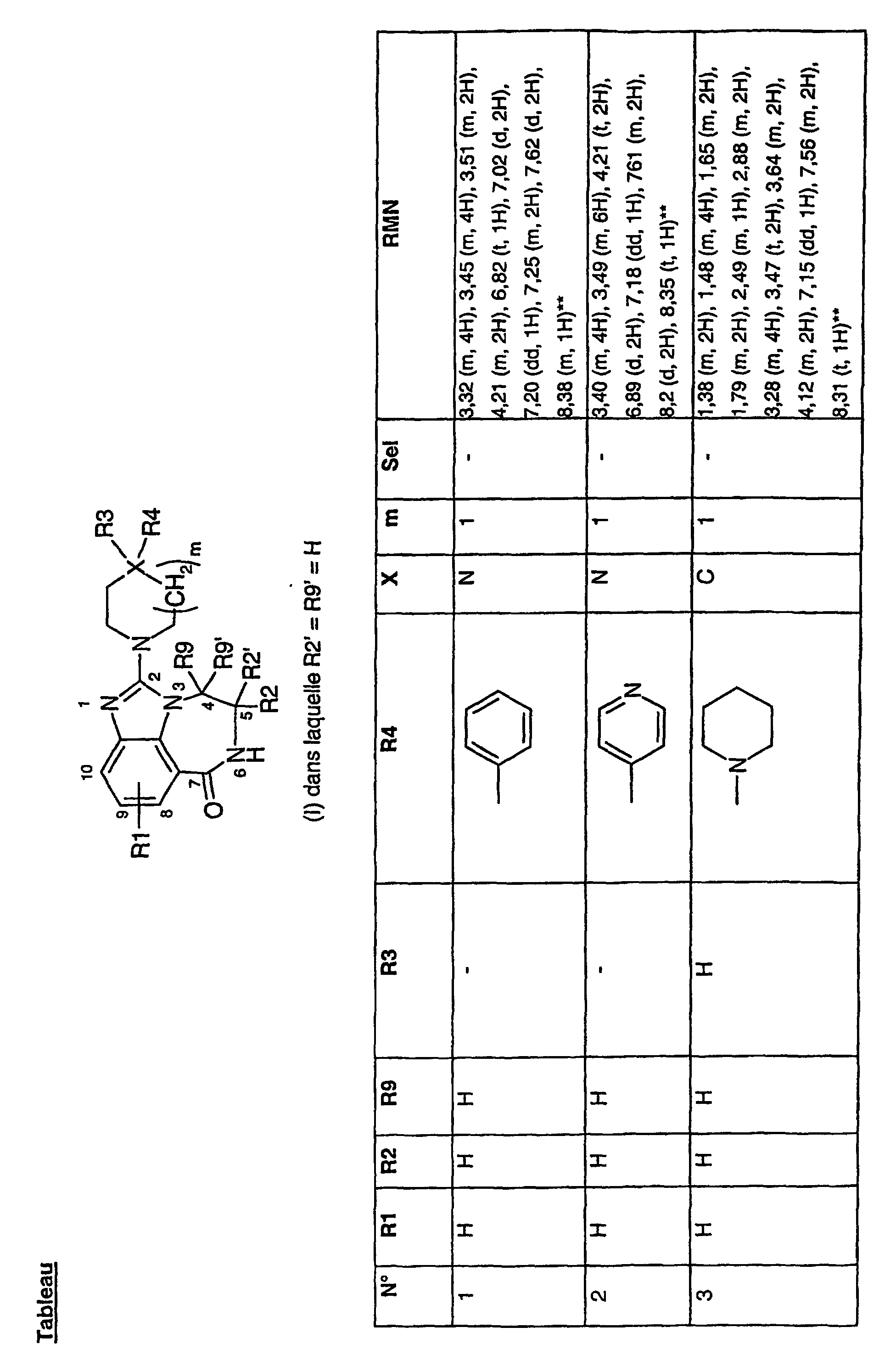

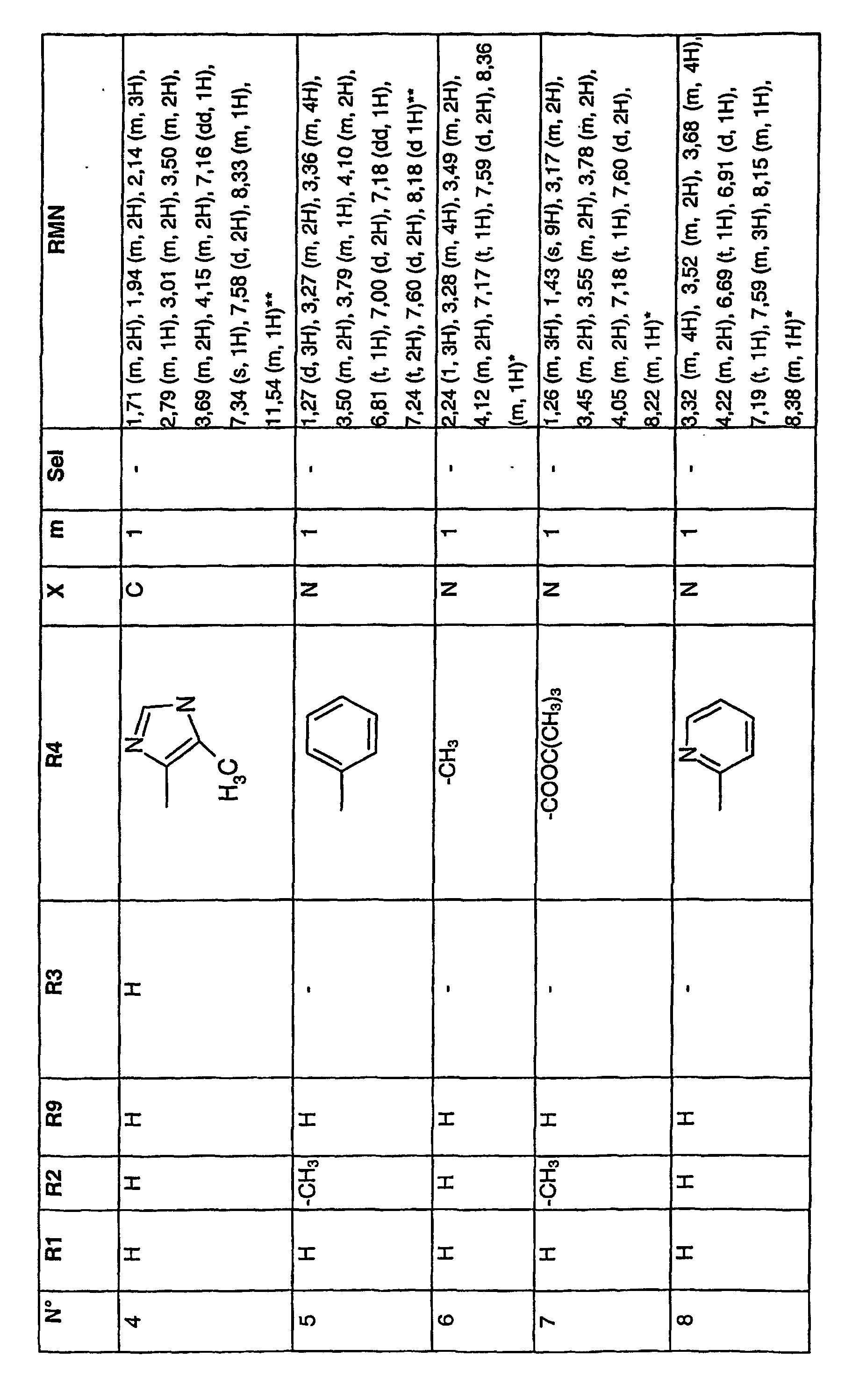

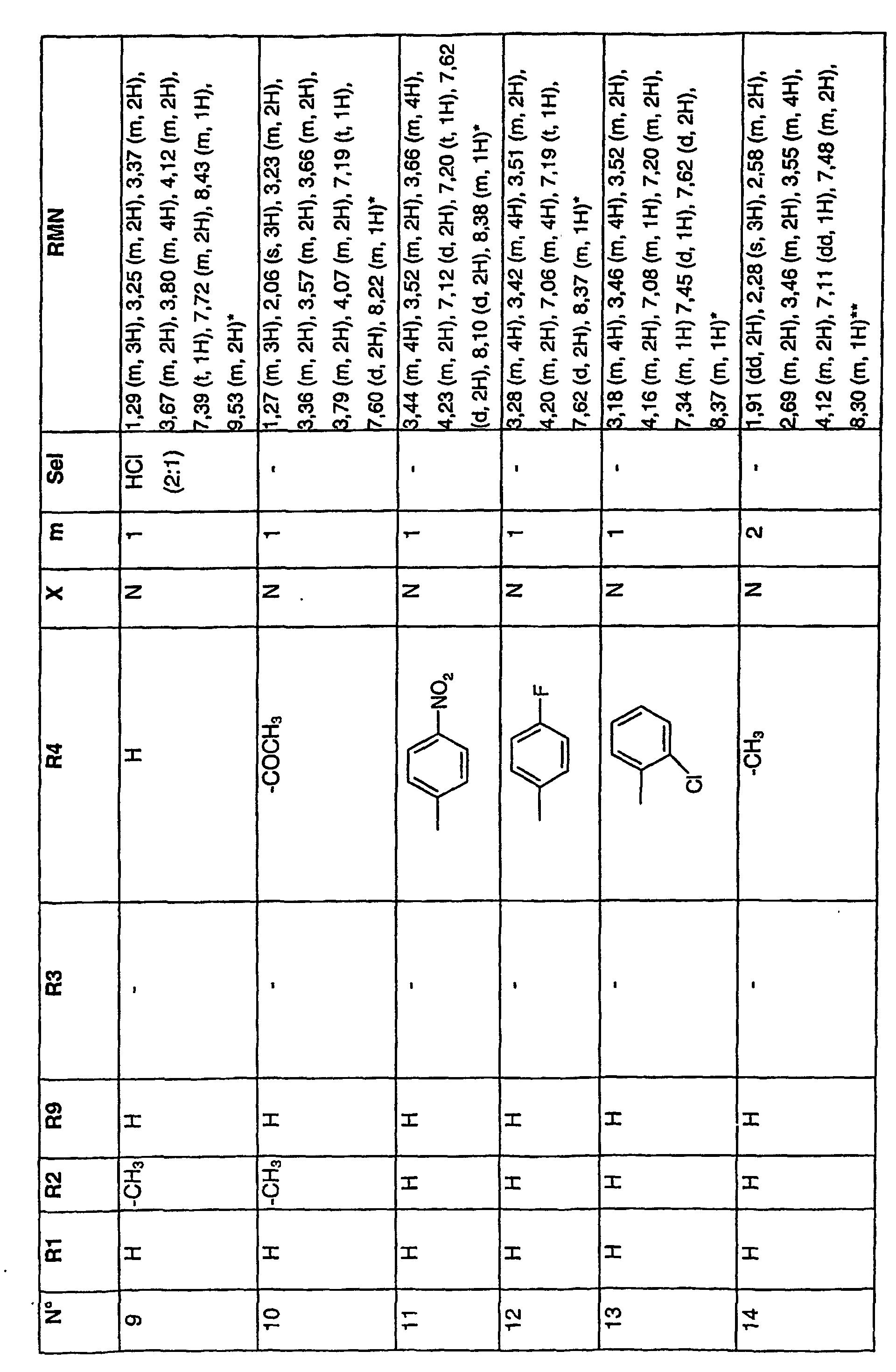

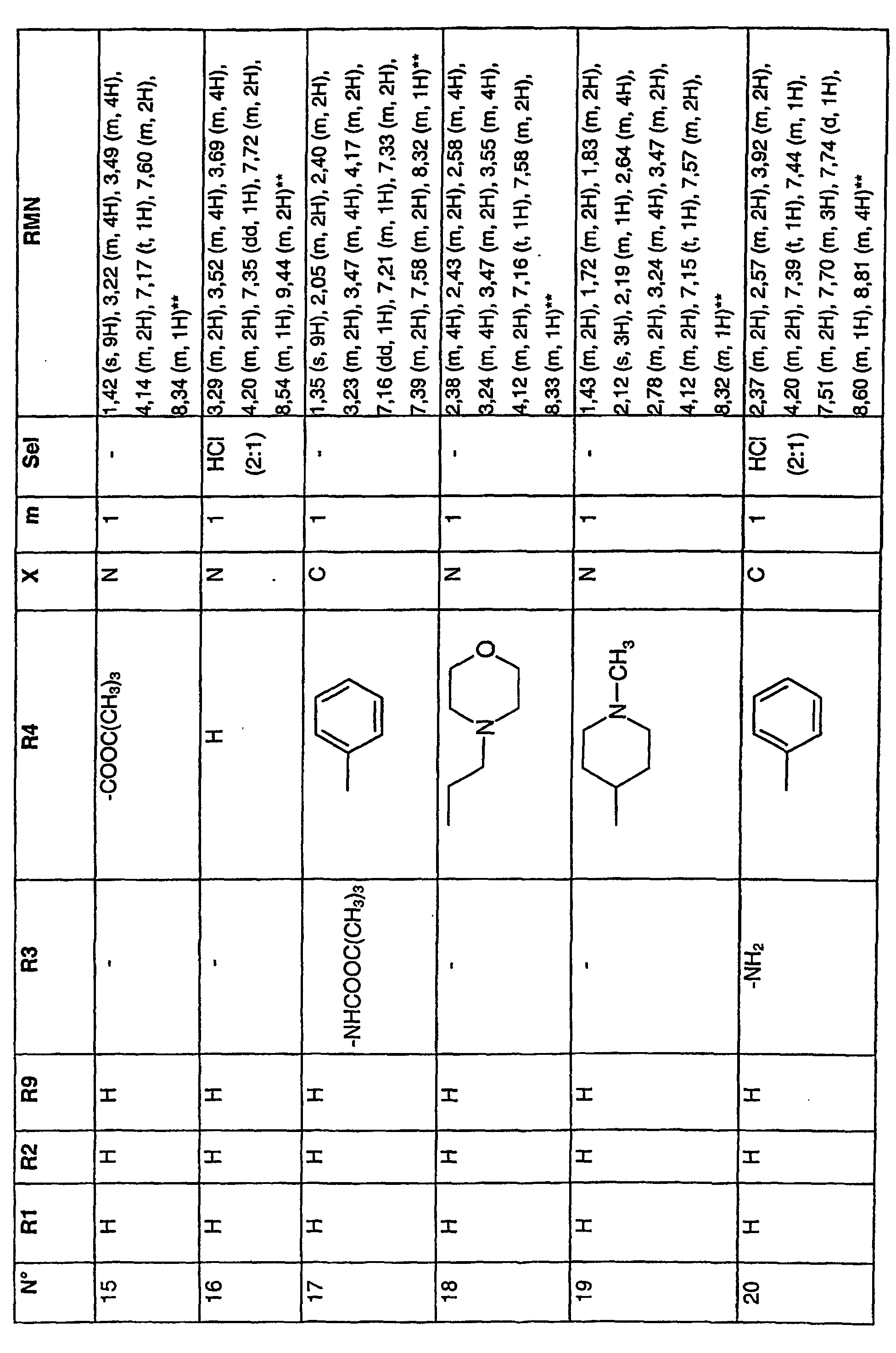

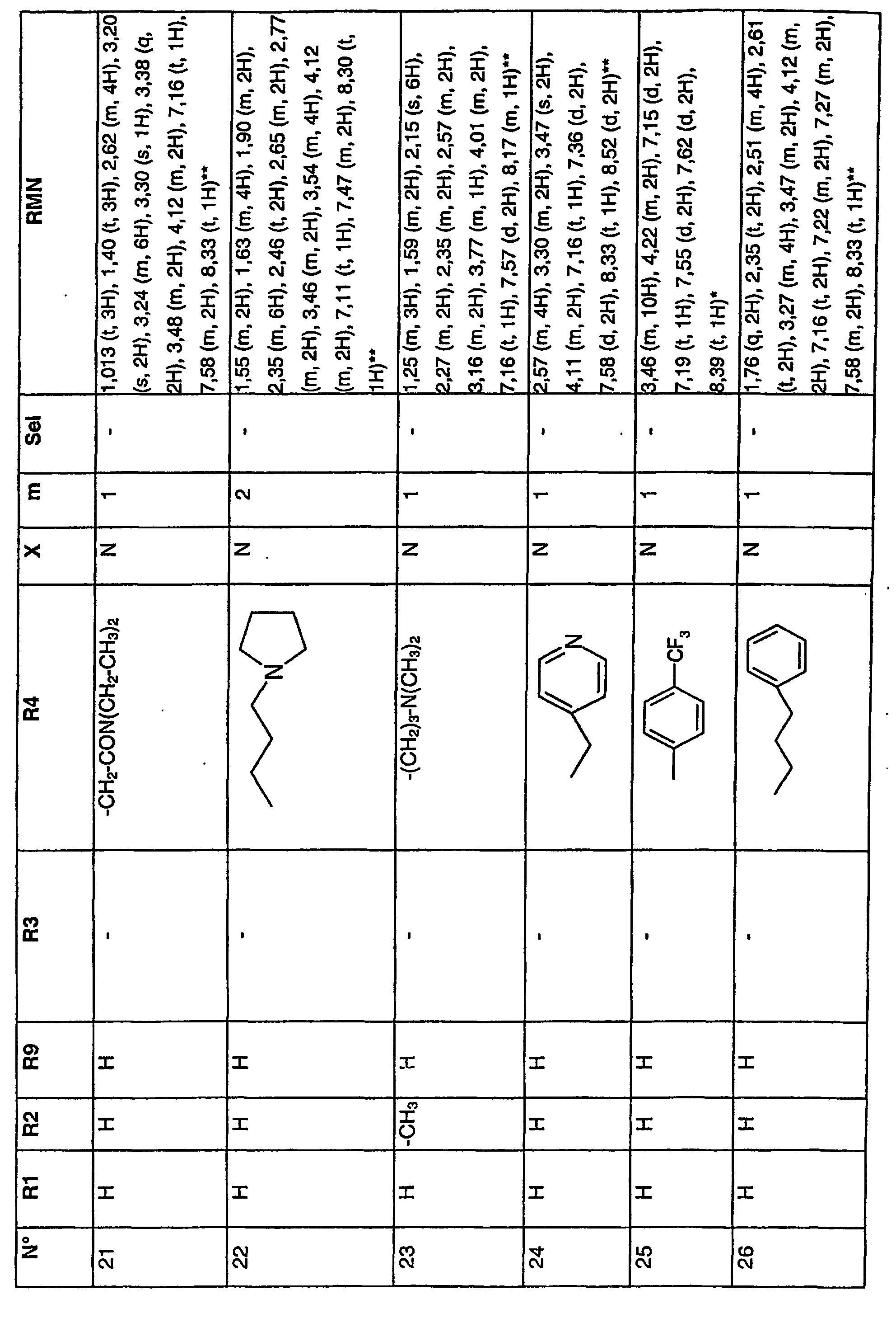

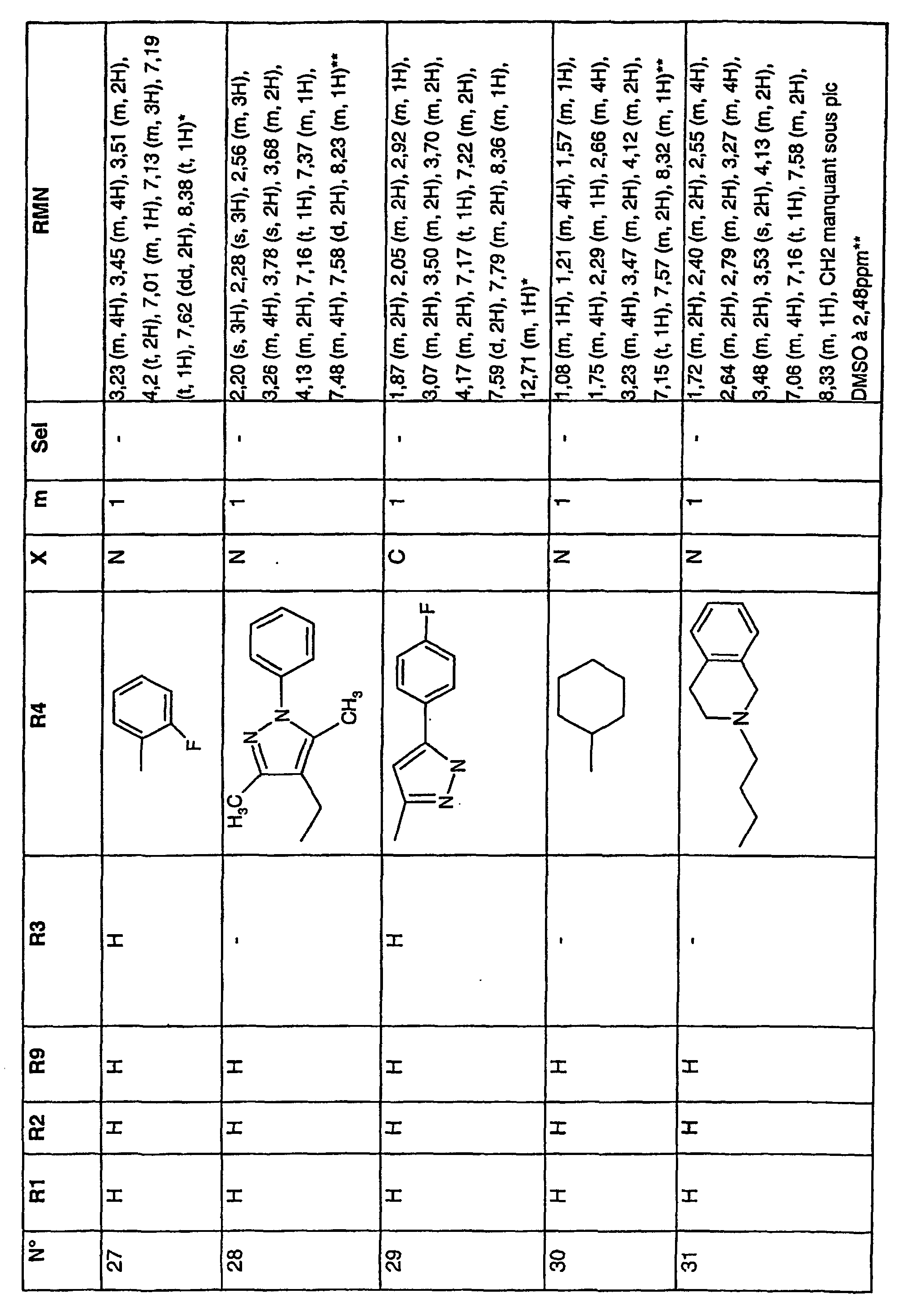

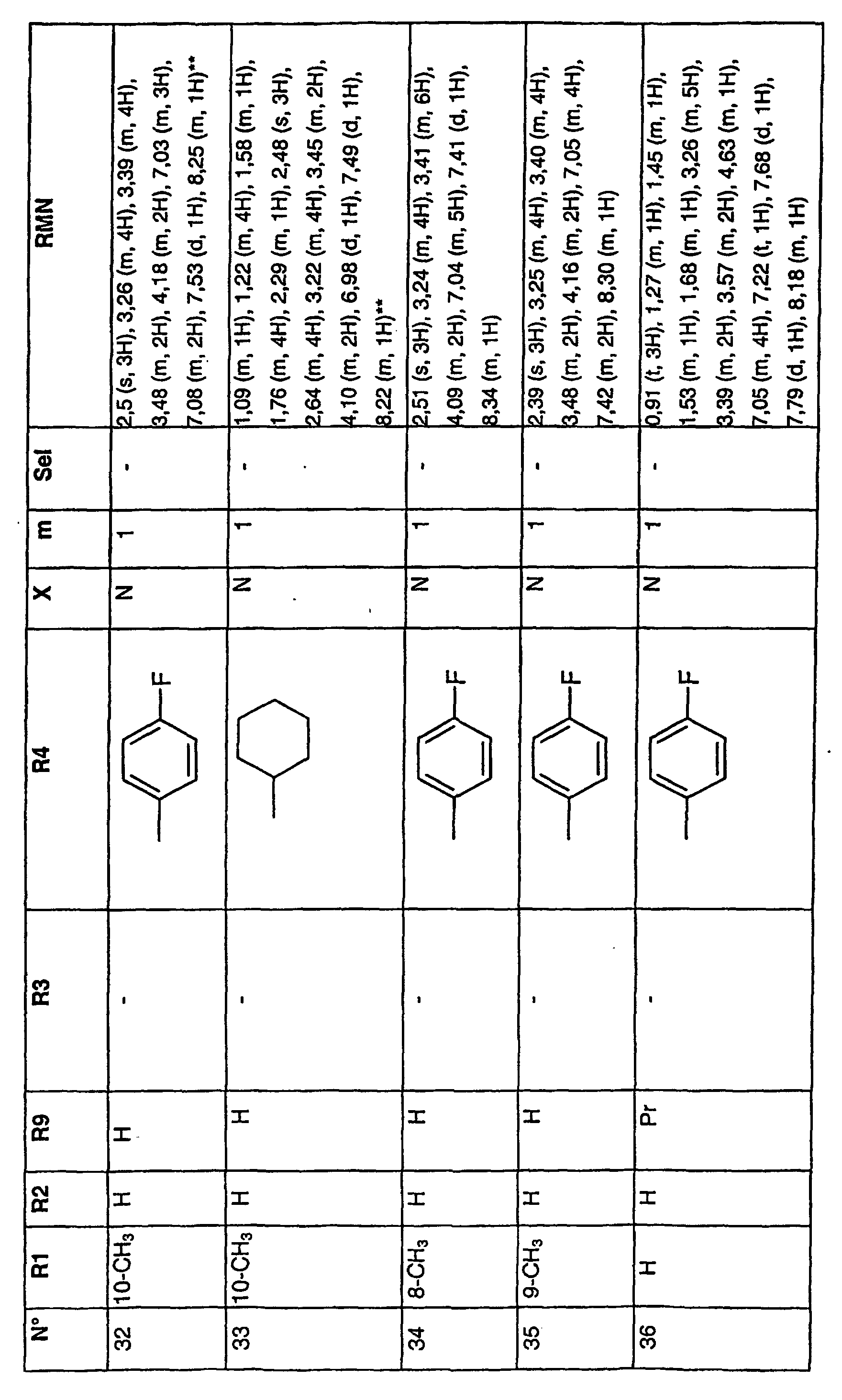

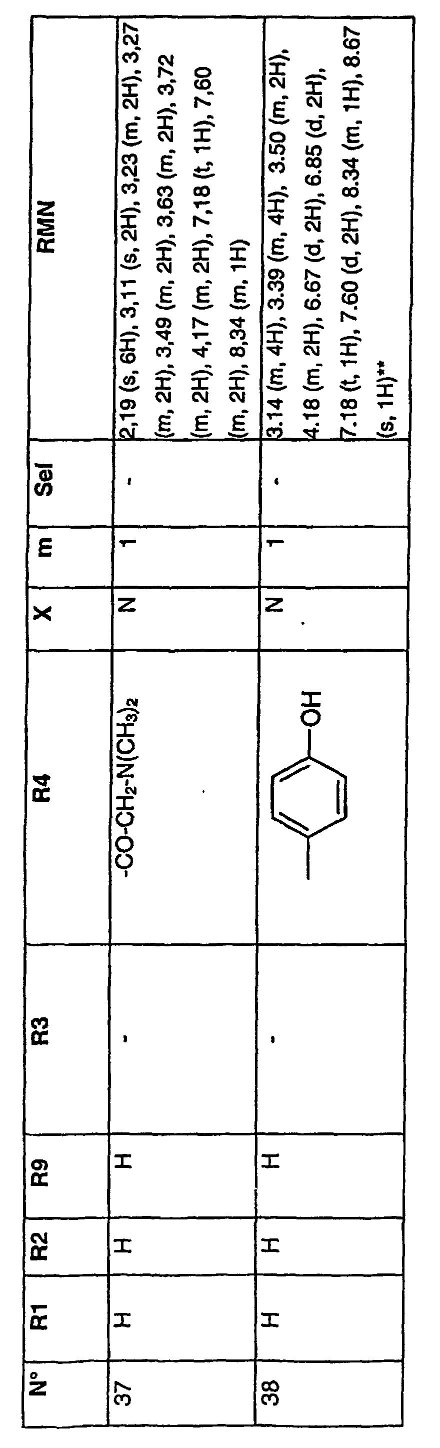

Les composés de l'invention ont fait l'objet d'essais pharmacologiques permettant de déterminer leur effet inhibiteur de la PARP ou poly(ADP-ribose)polymérase. Les composés de l'invention ont été soumis au test suivant :

### Effets des composés sur l'activité enzymatique de la PARP

La PARP-1 humaine recombinante (hPARP-1) est produite par les cellules *Sf9* en utilisant une système d'expression baculovirus (Giner *et al*., *Gene* (1992), **114**, 279-283). L'enzyme est partiellement purifiée à partir de l'extrait cellulaire obtenu après précipitation par le sulfate d'ammonium à 70%. La solution de hPARP-1 obtenue est capable de générer 0,5-0,7 nmol de nicotinamide à partir du NAD+ dans les conditions d'essai standard décrites ci-dessous. Les composés à tester sont mis en solution dans un milieu d'incubation contenant 50 mM de tris-HCl, 10 mM de MgCl₂, 20 µM d'acétate de zinc, 1,5 mM de dithiothreitol, 0,2 µg d'histone et 0,1 µg d'oligonucléotide (GGAATTCC) pour 100 µl, en présence de hPARP-1 partiellement purifiée tamponnée à pH 8. La réaction enzymatique est engagée par l'addition de NAD+ (0,2 mM) et poursuivie à température ambiante pendant 20 minutes. La réaction est stoppée par l'addition de HClO₄ (1,2 M) à 4°C. Après centrifugation, les supernageants sont analysés en HPLC (colonne Shandon Ultrabase C8). L'élution isocratique est réalisée par un tampon phosphate (0,1 M) de pH 4,5 contenant 6% d'acétonitrile, injecté à 1,25 ml/min pendant 6 minutes. Le nicotinamide formé est détecté en mesurant l'absorbance UV de l'éluat à 265 nm et quantifié par rapport au pic formé par un standard externe de nicotinamide (2 nmoles). L'activité hPARP-1 résiduelle mesurée en présence de concentrations variables de composés de l'invention est comparée à celle obtenue en leur absence. Toutes les mesures sont faites au moins en duplicate et les CI₅₀ sont calculées en utilisant l'équation de la sigmoïde de l'effet-dose.
Les composés les plus actifs dans cet essai se caractérisent par des CI₅₀ comprises entre 5 et 500 nM.

Par ailleurs, les composés conformes à l'invention les plus actifs en vers la PARP-2 se caractérisent également par des CI₅₀ comprises entre 5 et 500 nM.

Il apparaît donc que les composés de l'invention ont une activité inhibitrice sélective de la PARP, notamment de la PARP-1 et de la PARP-2.

Les composés de l'invention peuvent donc être utilisés pour la préparation de médicaments, en particulier de médicaments inhibiteurs de la PARP. Ces médicaments trouvent leur emploi en thérapeutique notamment dans la prévention ou le traitement de l'infarctus du myocarde, de l'ischémie cardiaque, l'insuffisance cardiaque, l'athérosclérose, la resténose après PTCA ou pontage, l'ischémie cérébrale et l'infarctus cérébral, causés par une ischémie, un traumatisme ou un accident thromboembolique, les maladies neurodégénératives comme la maladie de Parkinson, la maladie d'Alzheimer et la chorée de Huntington, l'insuffisance rénale aiguë, en particulier celle d'origine ischémique ou apparaissant après transplantation rénale, la transplantation cardiaque : traitement du rejet de greffe et de l'athérosclérose accélérée du greffon, les pathologies inflammatoires, les désordres immunologiques, les maladies rhumatoïdes, le diabète et les pancréatites, le choc septique, le syndrome de détresse respiratoire aiguë, les tumeurs et les métastases, les maladies autoimmunes, le SIDA, l'hépatite, le psoriasis, les vasculites, les colites ulcéreuses, les sclérose multiples et la myasthénie.

Ainsi, les composés de formule (I) conformes à l'invention peuvent être utilisés pour la préparation d'un médicament destiné au traitement ou à la prévention des désordres dans lesquels l'enzyme PARP est impliquée.

Enfin, la présente invention concerne également des compositions pharmaceutiques renfermant, en tant que principe actif, un composé de formule (I) selon l'invention. Ces compositions pharmaceutiques contiennent une dose efficace d'un composé selon l'invention, ou un sel pharmaceutiquement acceptable de ce dernier, et un ou plusieurs excipients pharmaceutiques convenables. Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intra-veineuse, topique, intratrachéale, intranasale, transdermique ou rectale, le principe actif de formule (I) ci-dessus, ou son sel ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades ou lotions.

La dose de principe actif administrée par jour peut atteindre 0,1 à 1000 mg/kg par voie orale, parentérale ou rectale. Il peut y avoir des cas particuliers où des dosages plus élevés ou plus faibles sont appropriés, de tels dosages ne sortent pas du cadre de l'invention. Selon la pratique habituelle, le dosage approprié à chaque patient est déterminé par le médecin selon le mode d'administration, le poids et la réponse dudit patient.

Lorsqu'on prépare une composition solide sous forme de comprimés, on mélange l'ingrédient actif principal avec un excipient pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières. Les comprimés peuvent être réalisés par différentes techniques, telles que la compression directe, la granulation sèche, la granulation humide ou la fusion à chaud.

On obtient une préparation sous forme de gélules en mélangeant l'ingrédient actif avec un diluant et en versant le mélange obtenu dans des gélules molles ou dures.

Pour une administration parentérale, on peut utiliser des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

La présente invention, selon un autre de ses aspects, concerne également une méthode de traitement des pathologies ci-dessus indiquées qui comprend l'administration, à un patient, d'une dose efficace d'un composé de formule (I) selon l'invention ou un de ses sels pharmaceutiquement acceptables.

## Revendications

1. Composés répondant à la formule (I) 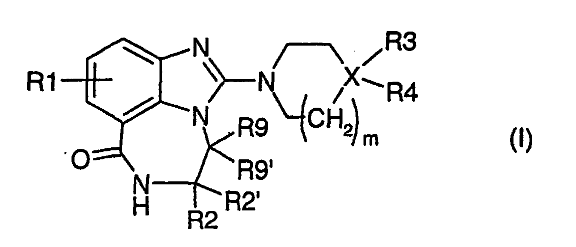 dans laquelle
**R1** représente un atome d'hydrogène, un groupe (C1-C4)alkyle, un atome d'halogène, un groupe nitro ou un groupe (C1-C4)alcoxy,
**R2, R2', R9** et **R9'** représentent indépendamment l'un de l'autre un atome d'hydrogène ou un groupe (C1-C4)alkyle,
**X** représente un atome d'azote ou un atome de carbone,
**m** est égal à 1 ou 2,
**et dans le cas où X représente un atome d'azote :**
**R3** représente un atome d'hydrogène ou un groupe (C1-C4)alkyle pour donner des composés de formule (I) comportant un ammonium quaternaire, ou encore n'existe pas pour donner des composés de formule (I) comportant une amine secondaire ou tertiaire,
R4 représente
un atome d'hydrogène,
un groupe (C1-C6)alkyle,
un groupe (C3-C7)cycloalkyle,
un groupe 4-pipéridyle éventuellement substitué, sur un atome de carbone ou sur l'atome d'azote, par un groupe (C1-C4)alkyle,
un groupe -(CH₂)ₚ-NR5R6, -(CH₂)ₚ-CONR5R6 ou -CO-(CH₂)ₚ-NR5R6, où p peut varier de 0 à 4 et où **R5** et **R6** représentent indépendamment l'un de l'autre un atome d'hydrogène ou un groupe (C1-C4)alkyle,
un groupe -(CH₂)ₚ-phényle, où p peut varier de 0 à 4 et où le noyau phényle est éventuellement substitué par un à trois groupes choisis indépendamment l'un de l'autre parmi : un groupe (C1-C4)alkyle, un groupe nitro, un groupe hydroxy, un groupe amino, un atome d'halogène, un groupe trifluorométhyle, un groupe (C1-C4)alcoxy, un groupe (C1-C4)alkylamino, un groupe (C1-C4)dialkylamino, un groupe -NHCHO, un groupe -NHCOR ou un groupe -NHSO₂R, où **R** représente un groupe (C1-C4)alcoxy ou un groupe (C1-C4)alkyle, ce groupe (C1-C4)alkyle pouvant être substitué par un groupe diméthylamino,
un groupe -(CH₂)ₚ-morpholinyle, -(CH₂)ₚ-pyrrolidinyle ou -(CH₂)ₚ-tétrahydro-isoquinoline, où p peut varier de 0 à 4,
un groupe -(CH₂)ₚ-hétéroaryle, où p peut varier de 0 à 4 et où le groupe hétéroaryle est choisi parmi les groupes pyridyle, aminopyridyle, pyridinyl-N-oxyde, pyrimidinyle, pyrazinyle, pyridazinyle, imidazolyle et pyrazolyle, ledit groupe hétéroaryle pouvant être substitué, sur un atome de carbone et/ou sur un atome d'azote, par un à trois groupes choisis parmi un groupe (C1-C4)alkyle ou un groupe phényle, ce groupe phényle pouvant lui-même être substitué par un à trois groupes choisis parmi les atomes d'halogène et les groupes (C1-C4)alkyles,
un groupe hétéroarylcarbonyle, le groupe hétéroaryle étant choisi parmi les groupes furyle, pyridyle, pyrimidyle, pyrazinyle, pyridazinyle et imidazolyle,
un groupe phénylcarbonyle, le groupe phényle pouvant éventuellement être substitué par un atome d'halogène,
un groupe (C1-C6)alkylcarbonyle,
un groupe -(CH₂)ₚ-COOR' où p peut varier de 0 à 4 et où R' représente un atome d'hydrogène ou un groupe (C1-C6)alkyle, ou bien
un groupe phénylsulfonyle éventuellement substitué sur le noyau phényle par un atome d'halogène, un groupe trifluorométhyle, un groupe (C1-C4)alkyle, un groupe nitro ou un groupe (C1-C4)alcoxy ;
**et dans le cas où X représente un atome de carbone :**
**R3** représente un atome d'hydrogène, un groupe -NR5R6, un groupe - NHCOR7, un groupe -CONHR5, un groupe -COR7, un groupe -NHCONH₂, un groupe -OH ou un groupe -CH₂OH,
**R4** représente :
un atome d'hydrogène,
un groupe -(CH₂)ₚ-phényle, où p peut varier de 0 à 4 et où le noyau phényle est éventuellement substitué par un à trois groupes choisis indépendamment l'un de l'autre parmi : un groupe (C1-C4)alkyle, un groupe nitro, un groupe hydroxy, un groupe amino, un atome d'halogène, un groupe trifluorométhyle, un groupe (C1-C4)alcoxy, un groupe (C1-C4)alkylamino, un groupe (C1-C4)dialkylamino, un groupe -NHCHO, un groupe -NHCOR ou un groupe -NHSO₂R, où **R** représente un groupe (C1-C4)alcoxy ou un groupe (C1-C4)alkyle, ce groupe (C1-C4)alkyle pouvant être substitué par un groupe diméthylamino,
un groupe -(CH₂)ₚ-hétéroaryle, où p peut varier de 0 à 4 et où le groupe hétéroaryle est choisi parmi les groupes pyridyle, aminopyridyle, pyridinyl-N-oxyde, pyrimidinyle, pyrazinyle, pyridazinyle, imidazolyle et pyrazolyle, ledit groupe hétéroaryle pouvant être substitué, sur un atome de carbone et/ou sur un atome d'azote, par un à trois groupes choisis parmi un groupe (C1-C4)alkyle ou un groupe phényle, ce groupe phényle pouvant lui-même être substitué par un à trois groupes choisis parmi les atomes d'halogène et les groupes (C1-C4)alkyles, ou bien
un groupe -(CH₂)ₜNR7R8, où t est égal à 0 ou 1,
**R5** et **R6** sont tels que définis ci-dessus,
**R7** et **R8** représentent indépendamment l'un de l'autre un groupe (C1-C4)alkyle ou (C1-C4)alcoxy, ou forment ensemble un cycle saturé comportant de 5 à 7 chaînons, comprenant éventuellement un atome d'azote supplémentaire, ce cycle pouvant être substitué par un groupe (C1-C4)alkyle sur un atome de carbone ou bien un atome d'azote, y compris sur l'atome d'azote sur lequel sont liés les groupes R7 et R8 pour former un ammonium quaternaire, parmi lesquels on peut citer un groupe pipéridin-1-yle, un groupe pyrrolidin-1-yle ou un groupe pipérazin-1-yle,
sous forme d'énantiomères, de diastéréoisomères ou de mélanges de ces différentes formes, y compris les mélanges racémiques, ainsi qu'à l'état de bases ou de sels d'addition à des acides pharmaceutiquement acceptables.

2. Composés de formule (I) selon la revendication 1, **caractérisés en ce que** :
**R1, R2** et **R9** représentent indépendamment l'un de l'autre un atome d'hydrogène ou un groupe (C1-C4)alkyle,
**R2'** et **R9'** représentent des atomes d'hydrogène,
**X** représente un atome d'azote,
**m** est égal à 1 ou 2,
**R3** représente un groupe (C1-C4)alkyle pour donner des composés de formule (I) comportant un ammonium quaternaire ou encore n'existe pas pour donner des composés de formule (I) comportant une amine secondaire ou tertiaire,
**R4** représente
un atome d'hydrogène,
un groupe (C1-C6)alkyle,
un groupe (C3-C7)cycloalkyle,
un groupe 4-pipéridyle éventuellement substitué, sur un atome de carbone ou sur l'atome d'azote, par un groupe (C1-C4)alkyle,
un groupe -(CH₂)ₚ-NR5R6, -(CH₂)ₚ-CONR5R6 ou -CO-(CH₂)ₚ-NR5R6, où p peut varier de 0 à 4 et où **R5** et **R6** représentent indépendamment l'un de l'autre un atome d'hydrogène ou un groupe (C1-C4)alkyle,
un groupe -(CH₂)ₚ-phényle, où p peut varier de 0 à 4 et où le noyau phényle est éventuellement substitué par un à trois groupes choisis indépendamment l'un de l'autre parmi un groupe nitro, un atome d'halogène et un groupe trifluorométhyle,
un groupe -(CH₂)ₚ-morpholinyle, -(CH₂)ₚ-pyrrolidinyle ou -(CH₂)ₚ-tétrahydro-isoquinoline, où p peut varier de 0 à 4,
un groupe -(CH₂)ₚ-pyrazolyle, où p peut varier de 0 à 4 et où le groupe pyrazolyle peut être substitué, sur un atome de carbone et/ou sur un atome d'azote, par un à trois groupes choisis parmi un groupe (C1-C4)alkyle ou un groupe phényle,
un groupe -(CH₂)ₚ-pyridyle, où p peut varier de 0 à 4,
un groupe hétéroarylcarbonyle, le groupe hétéroaryle étant choisi parmi un groupe furyle ou un groupe pyridyle,
un groupe phénylcarbonyle, le groupe phényle pouvant éventuellement être substitué par un atome d'halogène,
un groupe (C1-C6)alkylcarbonyle,
un groupe -(CH₂)ₚ-COOR' où p peut varier de 0 à 4 et où **R'** représente un groupe (C1-C6)alkyle, ou bien
un groupe phénylsulfonyle ;
Ou bien
**R1, R2'** et **R9'** représentent des atomes d'hydrogène,
**R2** et **R9** représentent indépendamment l'un de l'autre un atome d'hydrogène ou un groupe (C1-C4)alkyle,
**X** représente un atome de carbone,
**m** est égal à 1,
**R3** représente un atome d'hydrogène, un groupe -NR5R6, un groupe - NHCOR7, un groupe -CONHR5, un groupe -COR7, un groupe -NHCONH₂, un groupe -OH ou un groupe -CH₂OH,
**R4** représente
un atome d'hydrogène,
un groupe phényle,
un groupe hétéroaryle choisi parmi un groupe imidazolyle, éventuellement substitué par un groupe (C1-C4)alkyle, un groupe pyridyle, ou un groupe pyrazolyle éventuellement substitué, sur un atome d'azote ou sur un atome de carbone, par un groupe phényle pouvant lui-même être substitué par un à trois groupes choisis parmi les atomes d'halogène et les groupes (C1-C4)alkyles,
un groupe -NR7R8,
**R5** et **R6** représentent indépendamment l'un de l'autre un atome d'hydrogène ou un groupe (C1-C4)alkyle,
**R7** et **R8** représentent indépendamment l'un de l'autre un groupe (C1-C4)alkyle ou (C1-C4)alcoxy, ou forment ensemble un cycle saturé comportant de 5 à 7 chaînons, comprenant éventuellement un atome d'azote supplémentaire, ce cycle pouvant être substitué par un groupe (C1-C4)alkyle sur un atome de carbone ou bien un atome d'azote, y compris sur l'atome d'azote sur lequel sont liés les groupes R7 et R8 pour former un ammonium quaternaire, parmi lesquels on peut citer un groupe pipéridin-1-yle, un groupe pyrrolidin-1-yle ou un groupe pipérazin-1-yle,
sous forme d'énantiomères, de diastéréoisomères ou de mélanges de ces différentes formes, y compris les mélanges racémiques, ainsi qu'à l'état de bases ou de sels d'addition à des acides pharmaceutiquement acceptables.

3. Composés de formule (I) selon la revendication 1 ou la revendication 2, **caractérisés en ce que** :
**R1, R2** et **R9** représentent indépendamment l'un de l'autre un atome d'hydrogène ou un groupe (C1-C4)alkyle,
**R2'** et **R9'** représentent des atomes d'hydrogène,
**X** représente un atome d'azote,
**m** est égal à 1 ou 2,
**R3** représente un groupe (C1-C4)alkyle pour donner des composés de formule (I) comportant un ammonium quaternaire ou encore n'existe pas pour donner des composés de formule (I) comportant une amine secondaire ou tertiaire,
**R4** représente
un atome d'hydrogène,
un groupe (C1-C4)alkyle,
un groupe (C3-C7)cycloalkyle,
un groupe 4-pipéridyle éventuellement substitué sur l'atome d'azote par un groupe (C1-C4)alkyle,
un groupe -(CH₂)ₚ-NR5R6, -(CH₂)ₚ-CONR5R6 ou -CO-(CH₂)ₚ-NR5R6, où p est compris entre 1 et 3 et où **R5** et **R6** représentent indépendamment l'un de l'autre des groupes (C1-C4)alkyles,
un groupe -(CH₂)ₚ-phényle, où p peut varier de 0 à 4 et où le noyau phényle est éventuellement substitué par un à trois groupes choisis indépendamment l'un de l'autre parmi un groupe nitro, un atome d'halogène et un groupe trifluorométhyle,
un groupe -(CH₂)ₚ-morpholinyle, -(CH₂)ₚ-pyrrolidinyle ou -(CH₂)ₚ-tétrahydro-isoquinoline, où p est égal à 2 ou 3,
un groupe -(CH₂)ₚ-pyrazolyle, où p peut varier de 0 à 4 et où le groupe pyrazole peut être substitué, sur un atome de carbone et/ou sur un atome d'azote, par un à trois groupes choisis parmi un groupe (C1-C4)alkyle ou un groupe phényle,
un groupe -(CH₂)ₚ-hétéroaryle, où p est égal à 0 ou 1 et où le groupe hétéroaryle est un groupe 4-pyridyle ou 2-pyridyle,
un groupe (C1-C4)alkylcarbonyle, ou bien
un groupe -COOR' où **R'** représente un groupe (C1-C4)alkyle ;
Ou bien
**R1, R2'** et **R9'** représentent des atomes d'hydrogène,
**R2** et **R9** représentent indépendamment l'un de l'autre un atome d'hydrogène ou un groupe (C1-C4)alkyle,
**X** représente un atome de carbone,
**m** est égal à 1,
**R3** représente un atome d'hydrogène, un groupe -NH₂ ou un groupe - NHCOR7,
R4 représente
un groupe phényle,
un groupe hétéroaryle choisi parmi un groupe imidazolyle éventuellement substitué par un groupe (C1-C4)alkyle, ou un groupe pyrazolyle éventuellement substitué, sur un atome de carbone, par un groupe phényle pouvant lui-même être substitué par un à trois groupes choisis parmi les atomes d'halogène et les groupes (C1-C4)alkyles, ou bien
un groupe -NR7R8, où **R7** et **R8** représentent indépendamment l'un de l'autre un groupe (C1-C4)alkyle ou (C1-C4)alcoxy, ou forment ensemble un cycle saturé comportant de 5 à 7 chaînons, ce cycle pouvant être substitué par un groupe (C1-C4)alkyle sur un atome de carbone ou bien un atome d'azote, y compris sur l'atome d'azote sur lequel sont liés les groupes R7 et R8 pour former un ammonium quaternaire, tel qu'un groupe pipéridin-1-yle,
sous forme d'énantiomères, de diastéréoisomères ou de mélanges de ces différentes formes, y compris les mélanges racémiques, ainsi qu'à l'état de bases ou de sels d'addition à des acides pharmaceutiquement acceptables.

4. Composés de formule (I) selon la revendication 1, **caractérisés en ce que R1, R2, R2', R9, R9'** et m sont tels que définis dans la revendication 1 et **en ce que** :
**dans le cas où X représente un atome d'azote :**
**R3** est tel que défini dans la revendication **1**, et
**R4** représente :
un groupe 4-pipéridyle substitué, sur un atome de carbone ou sur l'atome d'azote, par un groupe (C1-C4)alkyle,
un groupe -(CH₂)ₚ-NR5R6 ou -(CH₂)ₚ-CONR5R6, où p peut varier de 1 à 4 et où R5 et R6 représentent indépendamment l'un de l'autre un groupe (C1-C4)alkyle,
un groupe -CO-(CH₂)ₚ-NR5R6, où p peut varier de 0 à 4 et où **R5** et **R6** représentent indépendamment l'un de l'autre un atome d'hydrogène ou un groupe (C1-C4)alkyle ;
un groupe -(CH₂)ₚ-phényle, où p peut varier de 1 à 4 et où le noyau phényle est éventuellement substitué par un à trois groupes choisis indépendamment l'un de l'autre parmi : un groupe (C1-C4)alkyle, un groupe nitro, un groupe hydroxy, un groupe amino, un atome d'halogène, un groupe trifluorométhyle, un groupe (C1-C4)alcoxy, un groupe (C1-C4)alkylamino, un groupe (C1-C4)dialkylamino, un groupe -NHCHO ou un groupe -NHCOR, où R représente un groupe (C1-C4)alcoxy ou un groupe (C1-C4)alkyle, ce groupe (C1-C4)alkyle pouvant être substitué par un groupe diméthylamino,
un groupe -(CH₂)ₚ-morpholinyle, -(CH₂)ₚ-pyrrolidinyle ou -(CH₂)ₚ-tétrahydro-isoquinoline, où p peut varier de 1 à 4,
un groupe -(CH₂)ₚ-hétéroaryle, où p peut varier de 1 à 4 et où le groupe hétéroaryle est choisi parmi les groupes pyridyle, aminopyridyle, pyrimidinyle, pyrazinyle, pyridazinyle, imidazolyle et pyrazolyle, ledit groupe hétéroaryle pouvant être substitué, sur un atome de carbone et/ou sur un atome d'azote, par un à trois groupes choisis parmi un groupe (C1-C4)alkyle ou un groupe phényle,
un groupe hétéroarylcarbonyle, le groupe hétéroaryle étant choisi parmi les groupes furyle, pyridyle, pyrimidyle, pyrazinyle, pyridazinyle et imidazolyle,
un groupe phénylcarbonyle, le groupe phényle pouvant éventuellement être substitué par un atome d'halogène,
un groupe (C1-C6)alkylcarbonyle,
un groupe -(CH₂)ₚ-COOR' où p peut varier de 0 à 4 et où **R'** représente un atome d'hydrogène ou un groupe (C1-C6)alkyle,
un groupe phénylsulfonyle éventuellement substitué sur le noyau phényle par un atome d'halogène, un groupe trifluorométhyle, un groupe (C1-C4)alkyle, un groupe nitro ou un groupe (C1-C4)alcoxy, ou bien
un groupe -CO-(CH₂)ₚ-NR5R6, où p peut *varier* de 0 à 4 et où R5 et R6 représentent indépendamment l'un de l'autre un atome d'hydrogène ou un groupe (C1-C4)alkyle ;
**et dans le cas où X représente un atome de carbone :**
• soit **R3** représente un groupe -NR5R6, où **R5** et **R6** représentent indépendamment l'un de l'autre un groupe (C1-C4)alkyle ; un groupe -NHCOR7, où R7 représente un groupe (C1-C4)alcoxy; un groupe -CONHR5, où R5 représente un atome d'hydrogène ou un groupe (C1-C4)alkyle; un groupe -COR7, où R7 représente un groupe (C1-C4)alkyle ou (C1-C4)alcoxy ; ou un groupe -NHCONH₂, **R4** étant tel que défini dans la revendication 1,
• soit **R3** est tel que défini dans la revendication 1 et **R4** représente :
un groupe phényle substitué par un à trois groupes choisis indépendamment l'un de l'autre parmi : un groupe (C1-C4)alkyle ou un groupe trifluorométhyle,
un groupe imidazolyle substitué par un groupe (C1-C4)alkyle,
un groupe pyrazolyle substitué, sur un atome d'azote ou sur un atome de carbone, par un groupe phényle pouvant lui-même être substitué par un à trois groupes choisis parmi les atomes d'halogène et les groupes (C1-C4)alkyles,
un groupe -CH₂-NR7R8, où **R7** et **R8** sont tels que définis dans la revendication 1, ou bien
un groupe -NR7R8, où **R7** et **R8** représentent indépendamment l'un de l'autre un groupe (C1-C4)alkyle ou forment ensemble un cycle saturé comportant de 5 à 7 chaînons, comprenant éventuellement un atome d'azote supplémentaire, ce cycle pouvant être substitué par un groupe (C1-C4)alkyle sur un atome de carbone ou bien un atome d'azote, y compris sur l'atome d'azote sur lequel sont liés les groupes R7 et R8 pour former un ammonium quaternaire, parmi lesquels on peut citer un groupe pipéridin-1-yle, un groupe pyrrolidin-1-yle ou un groupe pipérazin-1-yle,
sous forme d'énantiomères, de diastéréoisomères ou de mélanges de ces différentes formes, y compris les mélanges racémiques, ainsi qu'à l'état de bases ou de sels d'addition à des acides pharmaceutiquement acceptables.

5. Procédé de préparation des composés de formule (I) selon l'une quelconque des revendications 1 à 4, **caractérisé en ce que** l'on fait réagir un dérivé de formule (II), 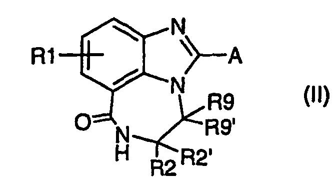 dans laquelle R1, R2, R2', R9 et R9' sont tels que définis dans la revendication 1 et A représente un groupe partant, en présence d'une amine de formule (III), 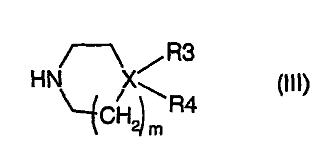 dans laquelle X, R3, R4 et m sont tels que définis dans la revendication 1, dans un solvant qui peut être un alcool, un éther, la diméthylformamide ou bien un hydrocarbure pour obtenir le composé de formule (I) selon la revendication 1, la réaction pouvant s'effectuer en présence d'une base, en présence d'halogénures de métal alcalin ou en présence de catalyseurs à base de palladium ou de nickel.

6. Composés de formule (II) telle que définie dans la revendication 5, utiles en tant qu'intermédiaires de synthèse.

7. Médicament, **caractérisé en ce qu'**il comprend un composé de formule (I) selon l'une quelconque des revendications 1 à 4.

8. Composition pharmaceutique, **caractérisée en ce qu'**elle comprend un composé de formule (I) selon l'une quelconque des revendications 1 à 4, ou un sel pharmaceutiquement acceptable de ce dernier, ainsi qu'un ou plusieurs excipients pharmaceutiques convenables.

9. Utilisation d'un composé de formule (I) selon l'une quelconque des revendications 1 à 4 pour la préparation d'un médicament destiné au traitement ou à la prévention des désordres dans lesquels l'enzyme poly(ADP-ribose)polymérase est impliquée.

## Patentansprüche

1. Verbindungen der Formel (I) 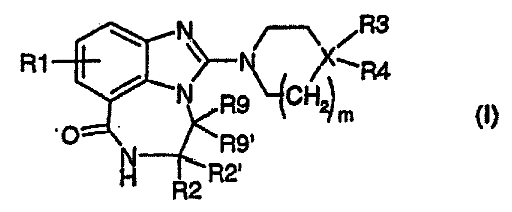 in der
**R1** ein Wasserstoffatom, eine (C1-C4)-Alkylgruppe, ein Halogenatom, eine Nitrogruppe oder eine C1-C4)-Alkoxygruppe bedeutet,
**R2, R2', R9** und **R9'** unabhängig voneinander ein Wasserstoffatom oder eine (C1-C4)-Alkylgruppe bedeuten,
**X** ein Stickstoffatom oder ein Kohlenstoffatom darstellt,
**m** 1 oder 2 bedeutet,
**und dann, wenn X ein Stickstoffatom darstellt:**
**R3** ein Wasserstoffatom oder eine (C1-C4)-Alkylgruppe bedeutet, zur Bildung von Verbindungen der Formel (I), die eine quaternäre Ammoniumgruppe aufweisen, oder nicht existiert zur Bildung von Verbindungen der Formel (I), die ein sekundäres oder tertiäres Amin aufweisen,
**R4**:
ein Wasserstoffatom,
eine (C1-C6)-Alkylgruppe.
eine C3-C7)-Cycloalkylgruppe,
eine 4-Piperidylgruppe, die gegebenenfalls an einem Kohlenstoffatom oder am Stickstoffatom durch eine (C1-C4)-Alkylgruppe substituiert ist,
eine Gruppe -(CH₂)ₚ-NR5R6, -(CH₂)ₚ-CONR5R6 oder -CO-(CH₂)ₚ-NR5R6, in denen p einen Wert von 0 bis 4 aufweisen kann und worin **R5** und **R6** unabhängig voneinander ein Wasserstoffatom oder eine (C1-C4)-Alkylgruppe darstellen,
eine -(CH₂)ₚ-Phenylgruppe, in der p den Wert 0 bis 4 aufweisen kann, und der Phenylkern gegebenenfalls durch eine bis drei Gruppen substituiert ist, unabhängig voneinander ausgewählt aus: einer (C1-C4)-Alkylgruppe, einer Nitrogruppe, einer Hydroxygruppe, einer Aminogruppe, einem Halogenatom, einer Trifluormethylgruppe, einer (C1-C4)-Alkoxygruppe, einer (C1-C4)-Alkylaminogruppe, einer (C1-C4)-Dialkylaminogruppe, einer Gruppe -NHCHO, einer Gruppe -NHCOR oder einer Gruppe -NHSO₂R, worin **R** eine (C1-C4)-Alkoxygruppe oder eine (C1-C4)-Alkylgruppe darstellt, wobei diese (C1-C4)-Alkylgruppe durch eine Dimethylaminogruppe substituiert sein kann, darstellt,
eine -(CH₂)ₚ-Morpholinylgruppe, -(CH₂)ₚ-Pyrrolidinylgruppe oder -(CH₂)ₚ-Tetrahydroisochinolingruppe, in denen p einen Wert von 0 bis 4 aufweisen kann,
eine -(CH₂)ₚ-Heteroarylgruppe, worin p einen Wert von 0 bis 4 aufweisen kann, und worin die Heteroarylgruppe ausgewählt ist aus Pyridyl-, Aminopyridyl-, Pyridinyl-N-oxid-, Pyrimidinyl-, Pyrazinyl-, Pyridazinyl-, Imidazolyl- und Pyrazolylgruppen, wobei die Heteroarylgruppe gegebenenfalls an einem Kohlenstoffatom und/oder einem Stickstoffatom durch eine bis drei Gruppen substituiert sein kann, ausgewählt aus einer (C1-C4)-Alkylgruppe oder einer Phenylgruppe, wobei diese Phenylgruppen ihrerseits durch eine bis drei Gruppen ausgewählt aus Halogenatomen und (C1-C4)-Alkylgruppen substituiert sein kann,
eine Heteroarylcarbonylgruppe, wobei die Heteroarylgruppe aus Furyl-, Pyridyl-, Pyrimidyl-, Pyrazinyl-, Pyridazinyl- und Imidazolylgruppen ausgewählt ist,
eine Phenylcarbonylgruppe, wobei die Phenylgruppe gegebenenfalls durch ein Halogenatom substituiert sein kann,
eine (C1-C6)-Alkylcarbonylgruppe,
eine Gruppe -(CH₂)ₚ-COOR', in der p den Wert von 0 bis 4 aufweisen kann und worin **R'** ein Wasserstoffatom oder eine (C1-C6)-Alkylgruppe darstellt, oder
eine Phenylsulfonylgruppe, die gegebenenfalls am Phenylkern durch ein Halogenatom, eine Trifluormethylgruppe, eine (C1-C4)-Alkylgruppe, eine Nitrogruppe oder eine (C1-C4)-Alkoxygruppe substituiert sein kann, bedeutet;
**und dann, wenn X ein Kohlenstoffatom darstellt:**
**R3** ein Wassestoffatom, eine Gruppe -NR5R6, eine Gruppe -NHCOR7, eine Gruppe -CONHR5, eine Gruppe -COR7, eine Gruppe -NHCONH₂, eine Gruppe - OH oder eine Gruppe -CH₂OH darstellt,
**R4**:
ein Wasserstoffatom,
eine -(CH₂)ₚ-Phenylgruppe, worin p einen Wert von 0 bis 4 aufweisen kann und wobei der Phenylkern gegebenenfalls durch eine bis drei Gruppen substituiert ist, die unabhängig voneinander ausgewählt sind aus: einer (C1-C4)-Alkylgruppe, einer Nitrogruppe, einer Hydroxygruppe, einer Aminogruppe, einem Halogenatom, einer Trifluormethylgruppe, einer (C1-C4)-Alkoxygruppe, einer (C1-C4)-Alkylaminogruppe, einer (C1-C4)-Dialkylaminogruppe, einer Gruppe -NHCHO, einer Gruppe -NHCOR oder einer Gruppe -NHSO₂R, worin **R** eine (C1-C4)-Alkoxygruppe oder eine (C1-C4)-Alkylgruppe darstellt, wobei diese (C1-C4)-Alkylgruppe durch eine Dimethylaminogruppe substituiert sein kann,
eine -(CH₂)ₚ-Heteroarylgruppe, worin p einen Wert von 0 bis 4 aufweisen kann, und worin die Heteroarylgruppe aus Pyridyl-, Aminopyridyl-, Pyridinyl-N-oxid-, Pyrimidinyl-, Pyrazinyl-, Pyridazinyl-, Imidazolyl- und Pyrazolylgruppen ausgewählt ist, wobei die Heteroarylgruppe an einem Kohlenstoffatom und/oder an einem Stickstoffatom durch eine bis drei Gruppen substituiert sein kann ausgewählt aus einer (C1-C4)-Alkylgruppe oder einer Phenylgruppe, wobei diese Phenylgruppe ihrerseits durch eine bis drei Gruppen ausgewählt aus Halogenatomen und (C1-C4)-Alkylgruppen substituiert sein kann, oder
eine Gruppe -(CH₂)ₜNR7R8, worin t den Wert 0 oder 1 besitzt, bedeutet,
**R5** und **R6** die oben angegebenen Bedeutungen besitzen,
**R7** und **R8** unabhängig voneinander eine (C1-C4)-Alkylgruppe oder (C1-C4)-Alkoxygruppe bedeuten oder gemeinsam einen gesättigten Ring mit 5 bis 7 Kettengliedern bilden, der gegebenenfalls ein zusätzliches Stickstoffatom enthalten kann, wobei dieser Ring durch eine (C1-C4)-Alkylgruppe substituiert sein kann, an einem Kohlenstoffatom oder an einem Stickstoffatom, einschließlich dem Stickstoffatom, an das die Gruppen R7 und R8 gebunden sind zur Bildung einer quaternären Ammoniumgruppe, als welche Gruppen man eine Piperidin-1-yl-gruppe, eine Pyrrolidin-1-yl-gruppe oder eine Piperazin-1-yl-gruppe nennen kann,
in Form der Enantiomeren, der Diastereoisomeren oder von Mischungen dieser verschiedenen Formen einschließlich der racemischen Mischungen sowie in Form der Basen oder von Additionssalzen mit pharmazeutisch annehmbaren Säuren.

2. Verbindungen der Formel (I) nach Anspruch 1, **dadurch gekennzeichnet, daß**:
**R1, R2** und **R9** unabhängig voneinander ein Wasserstoffatom oder eine (C1-C4)-Alkylgruppe bedeuten,
**R2'** und **R9'** Wasserstoffatome bedeuten,
**X** ein Stickstoffatom darstellt,
**m** 1 oder 2 bedeutet,
**R3** eine (C1-C4)-Alkylgruppe darstellt zur Bildung der Verbindungen der Formel (I), die eine quaternäre Ammoniumgruppe aufweisen, oder nicht vorhanden ist zur Bildung der Verbindungen der Formel (I), die ein sekundäres oder tertiäres Amin umfassen,
**R4**
ein Wasserstoffatom,
eine (C1-C6)-Alkylgruppe,
eine (C3-C7)-Cycloalkylgruppe,
eine 4-Piperidylgruppe, die gegebenenfalls an einem Kohlenstoffatom oder am Stickstoffatom durch eine (C1-C4)-Alkylgruppe substituiert ist,
eine Gruppe -(CH₂)ₚ-NR5R6, -(CH₂)ₚ-CONR5R6 oder -CO-(CH₂)ₚ-NR5R6, worin p einen Wert von 0 bis 4 aufweisen kann und worin **R5** und **R6** unabhängig voneinander ein Wasserstoffatom oder eine (C1-C4)-Alkylgruppe darstellen,
eine -(CH₂)ₚ-Phenylgruppe, worin p einen Wert 0 bis 4 aufweisen kann und worin der Phenylkern gegebenenfalls durch eine bis drei Gruppen substituiert ist, die unabhängig voneinander ausgewählt sind aus einer Nitrogruppe, einem Halogenatom und einer Trifluormethylgruppe,
eine -(CH₂)ₚ-Morpholinyl-, -(CH₂)ₚ-Pyrrolidinyl- oder -(CH₂)ₚ-Tetrahydroisochinolingruppe, worin p einen Wert von 0 bis 4 aufweisen kann,
eine -(CH₂)ₚ-Pyrazolylgruppe, worin p einen Wert von 0 bis 4 aufweisen kann und die Pyrazolylgruppe an einem Kohlenstoffatom und/oder an einem Stickstoffatom durch eine bis drei Gruppen ausgewählt aus einer (C1-C4)-Alkylgruppe oder einer Phenylgruppe substituiert sein kann,
eine -(CH₂)ₚ-Pyridylgruppe, worin p den Wert von 0 bis 4 aufweisen kann,
eine Heteroarylcarbonylgruppe, wobei die Heteroarylgruppe aus einer Furyloder einer Pyridylgruppe ausgewählt ist,
eine Phenylcarbonylgruppe, wobei die Phenylgruppe gegebenenfalls durch ein Halogenatom substituiert sein kann,
eine (C1-C6)-Alkylcarbonylgruppe,
eine -(CH₂)ₚ-COOR', worin p einen Wert von 0 bis 4 aufweisen kann und worin **R'** eine (C1-C6)-Alkylgruppe darstellt, oder
eine Phenylsulfonylgruppe bedeutet;
oder
**R1, R2'** und **R9'** Wasserstoffatome bedeuten,
**R2** und **R9** unabhängig voneinander ein Wasserstoffatom oder eine (C1-C4)-Alkylgruppe bedeuten,
**X** ein Kohlenstoffatom darstellt,
**m** den Wert 1 besitzt,
**R3** ein Wasserstoffatom, eine Gruppe -NR5R6, eine Gruppe -NHCOR7, eine Gruppe -CONHR5, eine Gruppe -COR7, eine Gruppe -NHCONH₂, eine Gruppe - OH oder eine Gruppe -CH₂OH bedeutet,
**R4**
ein Wasserstoffatom,
eine Phenylgruppe,
eine Heteroarylgruppe ausgewählt aus einer Imidazolylgruppe, die gegebenenfalls durch eine (C1-C4)-Alkylgruppe substituiert ist, eine Pyridylgruppe oder eine Pyrazolylgruppe, die gegebenenfalls an einem Stickstoffatom oder an einem Kohlenstoffatom durch eine Phenylgruppe substituiert ist, die ihrerseits durch eine bis drei Gruppen ausgewählt aus Halogenatomen und (C1-C4)-Alkylgruppen substituiert sein kann.
eine Gruppe -NR7R8 bedeutet,
**R5** und **R6** unabhängig voneinander ein Wasserstoffatom oder eine (C1-C4)-Alkylgruppe bedeuten,
**R7** und **R8** unabhängig voneinander eine (C1-C4)-Alkylgruppe oder (C1-C4)-Alkoxygruppe bedeuten oder gemeinsam einen gesättigten Ring mit 5 bis 7 Kettengliedern bilden, der gegebenenfalls ein zusätzliches Stickstoffatom enthält, wobei dieser Ring durch eine (C1-C4)-Alkylgruppe substituiert sein kann, an einem Kohlenstoffatom oder einem Stickstoffatom einschließlich dem Stickstoffatom, an das die Gruppen R7 und R8 gebunden sind zur Bildung einer quaternären Ammoniumgruppe, als welche Gruppen man eine Piperidin-1-yl-gruppe, eine Pyrrolidin-1-yl-gruppe oder eine Piperazin-1-yl-gruppe nennen kann,
in Form der Enantiomeren, der Diastereoisomeren oder von Mischungen dieser verschiedenen Formen einschließlich der racemischen Mischungen sowie in Form der Basen oder der Additionssalze mit pharmazeutisch annehmbaren Säuren.

3. Verbindungen der Formel (I) nach Anspruch 1 oder Anspruch 2, **dadurch gekennzeichnet, daß**:
**R1, R2** und **R9** unabhängig voneinander ein Wasserstoffatom oder eine (C1-C4)-Alkylgruppe bedeuten,
**R2'** und **R9'** Wasserstoffatome darstellen,
**X** ein Stickstoffatom bedeutet,
**m** 1 oder 2 bedeutet,
**R3** eine (C1-C4)-Alkylgruppe darstellt zur Bildung der Verbindungen der Formel (I), die eine quaternäre Ammoniumgruppe aufweisen, oder nicht vorliegt zur Bildung der Verbindungen der Formel (I), die eine sekundäre oder tertiäre Aminogruppe aufweisen,
**R4:**
ein Wasserstoffatom,
eine (C1-C4)-Alkylgruppe,
eine (C3-C7)-Cycloalkylgruppe,
eine 4-Piperidylgruppe, die gegebenenfalls am Stickstoffatom durch eine (C1-C4)-Alkylgruppe substituiert sein kann,
eine Gruppe -(CH₂)ₚ-NR5R6, -(CH₂)ₚ-CONR5R6 oder -CO-(CH₂)ₚ-NR5R6, worin p einen Wert zwischen 1 und 3 aufweist und worin **R5** und **R6** unabhängig voneinander (C1-C4)-Alkylgruppen darstellen,
eine -(CH₂)ₚ-Phenylgruppe, worin p einen Wert 0 bis 4 aufweisen kann und worin der Phenylkern gegebenenfalls durch eine bis drei Gruppen substituiert ist, die unabhängig voneinander ausgewählt sind aus einer Nitrogruppe, einem Halogenatom und einer Trifluormethylgruppe,
eine -(CH₂)ₚ-Morpholinyl-, -(CH₂)ₚ-Pyrrolidinyl- oder -(CH₂)ₚ-Tetrahydroisochinolingruppe, worin p den Wert 2 oder 3 besitzt,
eine -(CH₂)ₚ-Pyrazolylgruppe, worin p einen Wert von 0 bis 4 aufweisen kann und die Pyrazolgruppe an einem Kohlenstoffatom oder an einem Stickstoffatom durch eine bis drei Gruppen substituiert sein kann ausgewählt aus einer (C1-C4)-Alkylgruppe oder einer Phenylgruppe,
eine -(CH₂)ₚ-Heteroarylgruppe, worin p den Wert 0 oder 1 besitzt, und die Heteroarylgruppe eine 4-Pyridyl- oder 2-Pyridylgruppe darstellt,
eine (C1-C4)-Alkylcarbonylgruppe oder
eine Gruppe -COOR', worin **R'** eine (C1-C4)-Alkylgruppe darstellt, bedeutet;
oder
**R1, R2'** und **R9'** Wasserstoffatome bedeuten,
**R2** und **R9** unabhängig voneinander ein Wasserstoffatom oder eine (C1-C4)-Alkylgruppe darstellen,
**X** ein Kohlenstoffatom bedeutet,
**m** den Wert 1 besitzt,
**R3** ein Wasserstoffatom, eine Gruppe -NH₂ oder eine Gruppe -NHCOR7 darstellt,
**R4**:
eine Phenylgruppe,
eine Heteroarylgruppe ausgewählt aus einer Imidazolylgruppe, die gegebenenfalls durch eine (C1-C4)-Alkylgruppe substituiert ist, oder einer Pyrazolylgruppe, die gegebenenfalls an einem Kohlenstoffatom durch eine Phenylgruppe substituiert ist, die ihrerseits durch eine bis drei Gruppen ausgewählt aus Halogenatomen und (C1-C4)-Alkylgruppen substituiert sein kann, oder
eine Gruppe -NR7R8, worin **R7** und **R8** unabhängig voneinander eine (C1-C4)-Alkylgruppe oder (C1-C4)-Alkoxygruppe darstellen oder gemeinsam einen gesättigten Ring mit 5 bis 7 Kettengliedern bilden, welcher Ring durch eine (C1-C4)-Alkylgruppe substituiert sein kann, an einem Kohlenstoffatom oder einem Stickstoffatom einschließlich dem Stickstoffatom, an das die Gruppen R7 und R8 gebunden sind, zur Bildung einer quaternären Ammoniumgruppe, wie einer Piperidin-1-yl-gruppe, bedeutet,
in Form der Enantiomeren, der Diastereoisomeren oder von Mischungen dieser verschiedenen Formen einschließlich der racemischen Mischungen sowie in Form der Basen oder der Additionssalze mit pharmazeutisch annehmbaren Säuren.

4. Verbindungen der Formel (I) nach Anspruch 1, **dadurch gekennzeichnet, daß R1, R2, R2', R9, R9'** und **m** die in Anspruch 1 angegebenen Bedeutungen besitzen und daß:
**dann, wenn X ein Stickstoffatom darstellt:**
**R3** die in Anspruch 1 angegebenen Bedeutungen besitzt, und
**R4**:
eine 4-Piperidylgruppe, die an einem Kohlenstoffatom oder am Stickstoffatom durch eine (C1-C4)-Alkylgruppe substituiert ist,
eine Gruppe -(CH₂)ₚ-NR5R6 oder -(CH₂)ₚ-CONR5R6, worin p den Wert 1 bis 4 aufweisen kann und **R5** und **R6** unabhängig voneinander eine (C1-C4)-Alkylgruppe darstellen,
eine Gruppe -CO-(CH₂)ₚ-NR5R6, worin p einen Wert von 0 bis 4 aufweisen kann und worin **R5** und **R6** unabhängig voneinander ein Wassestoffatom oder eine (C1-C4)-Alkylgruppe bedeuten;
eine -(CH₂)ₚ-Phenylgruppe, worin p einen Wert 1 bis 4 aufweisen kann, und worin der Phenylkern gegebenenfalls durch eine bis drei Gruppen substituiert ist, die unabhängig voneinander ausgewählt sind aus: einer (C1-C4)-Alkylgruppe, einer Nitrogruppe, einer Hydroxygruppe, einer Aminogruppe, einem Halogenatom, einer Trifluormethylgruppe, einer (C1-C4)-Alkoxygruppe, einer (C1-C4)-Alkylaminogruppe, einer (C1-C4)-Dialkylaminogruppe, einer Gruppe -NHCHO oder einer Gruppe -NHCOR, worin **R** eine (C1-C4)-Alkoxygruppe oder eine (C1-C4)-Alkylgruppe darstellt, wobei diese (C1-C4)-Alkylgruppe durch eine Dimethylaminogruppe substituiert sein kann,
eine -(CH₂)ₚ-Morpholinyl-, -(CH₂)ₚ-Pyrrolidinyl- oder -(CH₂)ₚ-Tetrahydroisochinolingruppe, worin p einen Wert von 1 bis 4 aufweisen kann,
eine -(CH₂)ₚ-Heteroarylgruppe, worin p einen Wert von 1 bis 4 aufweisen kann, und worin die Heteroarylgruppe aus Pyridyl-, Aminopyridyl-, Pyrimidinyl-, Pyrazinyl-, Pyridazinyl-, Imidazolyl- und Pyrazolylgruppen ausgewählt ist, wobei diese Heteroarylgruppe an einem Kohlenstoffatom oder an einem Stickstoffatom durch eine bis drei Gruppen, ausgewählt aus einer (C1-C4)-Alkylgruppe oder einer Phenylgruppe substituiert sein kann,
eine Heteroarylcarbonylgruppe, wobei die Heteroarylgruppe aus Furyl-, Pyridyl-, Pyrimidyl-, Pyrazinyl-, Pyridazinyl- und Imidazolylgruppen ausgewählt ist,
eine Phenylcarbonylgruppe, wobei die Phenylgruppe gegebenenfalls durch ein Halogenatom substituiert sein kann,
eine (C1-C6)-Alkylcarbonylgruppe,
eine Gruppe -(CH₂)ₚ-COOR', worin p einen Wert von 0 bis 4 aufweisen kann und **R'** ein Wasserstoffatom oder eine (C1-C6)-Alkylgruppe bedeutet,
eine Phenylsulfonylgruppe, die gegebenenfalls am Phenylkern durch ein Halogenatom, eine Trifluormethylgruppe, eine (C1-C4)-Alkylgruppe, eine Nitrogruppe oder eine (C1-C4)-Alkoxygruppe substituiert sein kann, oder
eine Gruppe -CO-(CH₂)ₚ-NR5R6, worin p einen Wert von 0 bis 4 aufweisen kann und R5 und R6 unabhängig voneinander ein Wasserstoffatom oder eine (C1-C4)-Alkylgruppe darstellen, bedeutet;
**und dann, wenn X ein Kohlenstoffatom bedeutet:**
• entweder **R3** eine Gruppe -NR5R6 darstellt, worin R5 und R6 unabhängig voneinander eine (C1-C4)-Alkylgruppe darstellen; eine Gruppe -NHCOR7, worin **R7** eine (C1-C4)-Alkoxygruppe darstellt; eine Gruppe -CONHR5, worin **R5** ein Wasserstoffatom oder eine (C1-C4)-Alkylgruppe darstellt; eine Gruppe -COR7, worin **R7** eine (C1-C4)-Alkylgruppe oder (C1-C4)-Alkoxygruppe darstellt; oder eine Gruppe -NHCONH₂ bedeutet;
und **R4** die in Anspruch 1 angegebenen Bedeutungen besitzt,
• oder **R3** die in Anspruch 1 angegebenen Bedeutungen besitzt und **R4**:
eine Phenylgruppe, die durch eine bis drei Gruppen substituiert ist, die unabhängig voneinander aus einer (C1-C4)-Alkylgruppe oder einer Trifluormethylgruppe ausgewählt sind,
eine durch eine (C1-C4)-Alkylgruppe substituierte Imidazolylgruppe,
eine Pyrazolylgruppe, die an einem Stickstoffatom oder einem Kohlenstoffatom durch eine Phenylgruppe substituiert ist, die ihrerseits durch eine bis drei Gruppen ausgewählt aus Halogenatomen und (C1-C4)-Alkylgruppen substituiert sein kann,
eine Gruppe-CH₂-NR7R8, worin **R7** und **R8** die in Anspruch 1 angegebenen Bedeutungen besitzen, oder
eine Gruppe -NR7R8, worin **R7** und **R8** unabhängig voneinander eine (C1-C4)-Alkylgruppe bedeuten oder gemeinsam einen gesättigten Ring mit 5 bis 7 Kettengliedern bilden, der gegebenenfalls ein zusätzliches Stickstoffatom enthält, wobei dieser Ring durch eine (C1-C4)-Alkylgruppe substituiert sein kann an einem Kohlenstoffatom oder einem Stickstoffatom einschließlich dem Stickstoffatom, an welches die Gruppen R7 und R8 gebunden sind zur Bildung einer quaternären Ammoniumgruppe, worunter man eine Piperidin-1-yl-gruppe, eine Pyrrolidin-1-ylgruppe oder eine Piperazin-1-yl-gruppe nennen kann,
in Form der Enantiomeren, der Diastereoisomeren oder von Mischungen dieser verschiedenen Formen einschließlich der racemischen Mischungen sowie in Form der Basen oder der Additionssalze mit pharmazeutisch annehmbaren Säuren.

5. Verfahren zur Herstellung der Verbindungen der Formel (I) nach einem der Ansprüche 1 bis 4, **dadurch gekennzeichnet, daß** man ein Derivat der Formel (II): 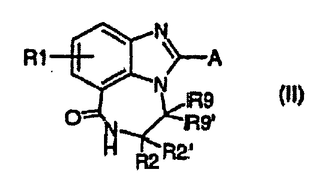 in der R1, R2, R2', R9 und R9' die in Anspruch 1 angegebenen Bedeutungen besitzen und A eine austretende Gruppe bedeutet, in Gegenwart eines Amins der Formel (III): 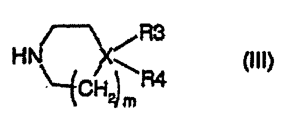 in der X, R3, R4 und m die in Anspruch 1 angegebenen Bedeutungen besitzen, in einem Lösungsmittel, welches ein Alkohol, ein Ether, Dimethylformamid oder ein Kohlenwasserstoff sein kann, umsetzt zur Bildung der Verbindung der Formel (I) nach Anspruch 1, wobei die Reaktion in Gegenwart einer Base, in Gegenwart von Alkalimetallhalogeniden oder in Gegenwart von Katalysatoren auf der Grundlage von Palladium oder Nickel durchgeführt werden kann.

6. Verbindungen der Formel (II) nach Anspruch 5, nützlich als Synthesezwischenprodukte.

7. Arzneimittel, **dadurch gekennzeichnet, daß** es eine Verbindung der Formel (I) nach einem der Ansprüche 1 bis 4 enthält.

8. Pharmazeutische Zubereitung, **dadurch gekennzeichnet, daß** sie eine Verbindung der Formel (I) nach einem der Ansprüche 1 bis 4 oder ein pharmazeutisch annehmbares Salz davon sowie ein oder mehrere pharmazeutisch annehmbare Trägermaterialien enthält.

9. Verwendung einer Verbindung der Formel (I) nach einem der Ansprüch 1 bis 4 zur Herstellung eines Arzneimittels zur Behandlung oder vorbeugenden Behandlung von Störungen, bei denen das Enzym Poly(ADP-ribose)-polymerase eine Rolle spielt.

## Claims

1. Compounds corresponding to formula (I) 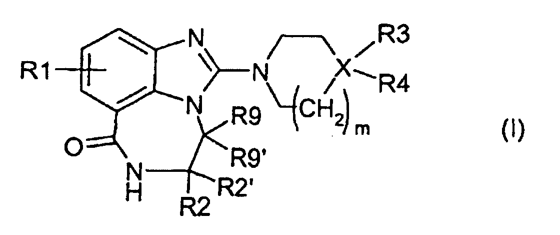 in which
**R1** represents a hydrogen atom, a (C1-C4)alkyl group, a halogen atom, a nitro group or a (C1-C4)alkoxy group,
**R2, R2', R9** and **R9'** represent, independently of each other, a hydrogen atom or a (C1-C4)alkyl group,
**X** represents a nitrogen atom or a carbon atom,
**m** is equal to 1 or 2,
**and when X represents a nitrogen atom:**
**R3** represents a hydrogen atom or a (C1-C4)alkyl group to give compounds of formula (I) comprising a quaternary ammonium, or alternatively it does not exist, to give compounds of formula (I) comprising a secondary or tertiary amine,
**R4** represents
a hydrogen atom,
a (C1-C6)alkyl group,
a (C3-C7)cycloalkyl group,
a 4-piperidyl group optionally substituted on a carbon atom or on the nitrogen atom with a (C1-C4)alkyl group,
a group -(CH₂)ₚ-NR5R6, -(CH₂)ₚ-CONR5R6 or -CO-(CH₂)ₚ-NR5R6, in which p may range from 0 to 4 and in which **R5** and **R6** represent, independently of each other, a hydrogen atom or a (C1-C4)alkyl group,
a group -(CH₂)ₚ-phenyl, in which p may range from 0 to 4 and in which the phenyl nucleus is optionally substituted with one to three groups chosen, independently of each other, from: a (C1-C4)alkyl group, a nitro group, a hydroxyl group, an amino group, a halogen atom, a trifluoromethyl group, a (C1-C4)alkoxy group, a (Cl-C4)alkylamino group, a (C1-C4)dialkylamino group, an -NHCHO group, a group - NHCOR or a group -NHSO₂R, in which **R** represents a (C1-C4)alkoxy group or a (C1-C4)alkyl group, this (C1-C4)alkyl group possibly being substituted with a dimethylamino group,
a group -(CH₂)ₚ-morpholinyl, -(CH₂)ₚ-pyrrolidinyl or -(CH₂)ₚ-tetrahydroisoquinoline, in which p may range from 0 to 4,
a group -(CH₂)ₚ-heteroaryl, in which p may range from 0 to 4 and in which the heteroaryl group is chosen from pyridyl, aminopyridyl, pyridyl N-oxide, pyrimidinyl, pyrazinyl, pyridazinyl, imidazolyl and pyrazolyl groups, the said heteroaryl group possibly being substituted, on a carbon atom and/or on a nitrogen atom, with one to three groups chosen from a (C1-C4)alkyl group or a phenyl group, this phenyl group itself possibly being substituted with one to three groups chosen from halogen atoms and (C1-C4)alkyl groups,
a heteroarylcarbonyl group, the heteroaryl group being chosen from furyl, pyridyl, pyrimidyl, pyrazinyl, pyridazinyl and imidazolyl groups,
a phenylcarbonyl group, the phenyl group possibly being substituted with a halogen atom,
a (C1-C6)alkylcarbonyl group,
a group -(CH₂)ₚ-COOR' in which p may range from 0 to 4 and in which **R'** represents a hydrogen atom or a (C1-C6)alkyl group, or alternatively
a phenylsulphonyl group optionally substituted on the phenyl nucleus with a halogen atom, a trifluoromethyl group, a (C1-C4)alkyl group, a nitro group or a (C1-C4)alkoxy group;
**and when X represents a carbon atom:**
**R3** represents a hydrogen atom, a group - NR5R6, a group -NHCOR7, a group -CONHR5, a group - COR7, an -NHCONH₂ group, an -OH group or a -CH₂OH group,
R4 represents:
a hydrogen atom,
a group -(CH₂)ₚ-phenyl, in which p may range from 0 to 4 and in which the phenyl nucleus is optionally substituted with one to three groups chosen, independently of each other, from: a (C1-C4)alkyl group, a nitro group, a hydroxyl group, an amino group, a halogen atom, a trifluoromethyl group, a (C1-C4)alkoxy group, a (C1-C4)alkylamino group, a (C1-C4)dialkylamino group, an -NHCHO group, a group - NHCOR or a group -NHSO₂R, in which **R** represents a (C1-C4)alkoxy group or a (C1-C4)alkyl group, this (C1-C4)alkyl group possibly being substituted with a dimethylaminc group,
a group -(CH₂)ₚ-heteroaryl, in which p may range from 0 to 4 and in which the heteroaryl group is chosen from pyridyl, aminopyridyl, pyridyl N-oxide, pyrimidinyl, pyrazinyl, pyridazinyl, imidazolyl and pyrazolyl groups, the said heteroaryl group possibly being substituted, on a carbon atom and/or on a nitrogen atom, with one to three groups chosen from a (C1-C4)alkyl group or a phenyl group, this phenyl group itself possibly being substituted with one to three groups chosen from halogen atoms and (C1-C4)alkyl groups, or alternatively
a group -(CH₂)ₜNR7R8, in which t is equal to 0 or 1,
**R5** and **R6** are as defined above,
**R7** and **R8** represent, independently of each other, a (C1-C4)alkyl or (C1-C4)alkoxy group, or together form a 5- to 7-membered saturated ring optionally comprising an additional nitrogen atom, this ring possibly being substituted with a (C1-C4)alkyl group on a carbon atom or a nitrogen atom, including on the nitrogen atom to which the groups R7 and R8 are attached to form a quaternary ammonium, among which mention may be made of a 1-piperidyl group, a 1-pyrrolidinyl group or a 1-piperazinyl group,
in the form of enantiomers, diastereoisomers or mixtures of these various forms, including racemic mixtures, and also in the form of bases or addition salts with pharmaceutically acceptable acids.

2. Compounds of formula (I) according to Claim 1, **characterized in that**:
**R1, R2** and **R9** represent, independently of each other, a hydrogen atom or a (C1-C4)alkyl group,
**R2'** and **R9'** represent hydrogen atoms,
**X** represents a nitrogen atom,
**m** is equal to 1 or 2,
**R3** represents a (C1-C4)alkyl group to give compounds of formula (I) comprising a quaternary ammonium, or alternatively it does not exist, to give compounds of formula (I) comprising a secondary or tertiary amine,
**R4** represents
a hydrogen atom,
a (C1-C6)alkyl group,
a (C3-C7)cycloalkyl group,
a 4-piperidyl group, optionally substituted on a carbon atom or on the nitrogen atom with a (C1-C4)alkyl group,
a group -(CH₂)ₚ-NR5R6, - (CH₂)ₚ-CONR5R6 or - CO-(CH₂)ₚ-NR5R6, in which p may range from 0 to 4 and in which **R5** and **R6** represent, independently of each other, a hydrogen atom or a (C1-C4)alkyl group,
a group -(CH₂)ₚ-phenyl, in which p may range from 0 to 4 and in which the phenyl nucleus is optionally substituted with one to three groups chosen, independently of each other, from a nitro group, a halogen atom and a trifluoromethyl group,
a group - (CH₂)ₚ-morpholinyl, - (CH₂)ₚ-pyrrolidinyl or -(CH₂)ₚ-tetrahydroisoquinoline, in which p may range from 0 to 4,
a group -(CH₂)ₚ-pyrazolyl, in which p may range from 0 to 4 and in which the pyrazolyl group may be substituted, on a carbon atom and/or on a nitrogen atom, with one to three groups chosen from a (C1-C4)alkyl group or a phenyl group,
a group -(CH₂)ₚ-pyridyl, in which p may range from 0 to 4,
a heteroarylcarbonyl group, the heteroaryl group being chosen from a furyl group or a pyridyl group,
a phenylcarbonyl group, the phenyl group possibly being substituted with a halogen atom,
a (C1-C6)alkylcarbonyl group,
a group -(CH₂)ₚ-COOR' in which p may range from 0 to 4 and in which **R'** represents a (C1-C6)alkyl group, or
a phenylsulphonyl group;
or where
**R1, R2'** and **R9'** represent hydrogen atoms,
**R2** and **R9** represent, independently of each other, a hydrogen atom or a (C1-C4)alkyl group,
**X** represents a carbon atom,
**m** is equal to 1,
**R3** represents a hydrogen atom, a group - NR5R6, a group -NHCOR7, a group -CONHR5, a group - COR7, an -NHCONH₂ group, an -OH group or a -CH₂OH group,
**R4** represents
a hydrogen atom,
a phenyl group,
a heteroaryl group chosen from an imidazolyl group, optionally substituted with a (C1-C4)alkyl group, a pyridyl group or a pyrazolyl group, optionally substituted on a nitrogen atom or on a carbon atom with a phenyl group which may itself be substituted with one to three groups chosen from halogen atoms and (C1-C4)alkyl groups,
a group -NR7R8,
**R5** and **R6** represent, independently of each other, a hydrogen atom or a (C1-C4)alkyl group,
**R7** and **R8** represent, independently of each other, a (C1-C4)alkyl or (C1-C4)alkoxy group, or together form a saturated 5- to 7-membered ring optionally comprising an additional nitrogen atom, this ring possibly being substituted with a (C1-C4)alkyl group on a carbon atom or on a nitrogen atom, including on the nitrogen atom to which the groups R7 and R8 are attached to form a quaternary ammonium, among which mention may be made of a 1-piperidyl group, a 1-pyrrolidinyl group or a 1-piperazinyl group,
in the form of enantiomers, diastereoisomers or mixtures of these various forms, including racemic mixtures, and also in the form of bases or addition salts with pharmaceutically acceptable acids.

3. Compounds of formula (I) according to Claim 1 or Claim 2, **characterized in that**:
**R1, R2** and **R9** represent, independently of each other, a hydrogen atom or a (C1-C4)alkyl group,
**R2'** and **R9'** represent hydrogen atoms,
**X** represents a nitrogen atom,
**m** is equal to 1 or 2,
**R3** represents a (C1-C4)alkyl group, to give compounds of formula (I) comprising a quaternary ammonium, or alternatively it does not exist, to give compounds of formula (I) comprising a secondary or tertiary amine,
**R4** represents
a hydrogen atom,
a (C1-C4)alkyl group,
a (C3-C7)cycloalkyl group,
a 4-piperidyl group optionally substituted on the nitrogen atom with a (C1-C4)alkyl group,
a group -(CH₂)ₚ-NR5R6, -(CH₂)ₚ-CONR5R6 or - CO-(CH₂)ₚ-NR5R6, in which p is between 1 and 3 and in which **R5** and **R6** represent, independently of each other, (C1-C4)alkyl groups,
a group -(CH₂)ₚ-phenyl, in which p may range from 0 to 4 and in which the phenyl nucleus is optionally substituted with one to three groups chosen, independently of each other, from a nitro group, a halogen atom and a trifluoromethyl group,
a group -(CH₂)ₚ-morpholinyl, -(CH₂)ₚ-pyrrolidinyl or -(CH₂)ₚ-tetrahydroisoquinoline, in which p is equal to 2 or 3,
a group -(CH₂)ₚ-pyrazolyl, in which p may range from 0 to 4 and in which the pyrazole group may be substituted, on a carbon atom and/or on a nitrogen atom, with one to three groups chosen from a (C1-C4)alkyl group or a phenyl group,
a group -(CH₂)ₚ-heteroaryl, in which p is equal to 0 or 1 and in which the heteroaryl group is a 4-pyridyl or 2-pyridyl group,
a (C1-C4)alkylcarbonyl group, or
a group -COOR' in which **R'** represents a (C1-C4)alkyl group;
or where
**R1, R2'** and **R9'** represent hydrogen atoms,
**R2** and **R9** represent, independently of each other, a hydrogen atom or a (C1-C4)alkyl group,
**X** represents a carbon atom,
**m** is equal to 1,
**R3** represents a hydrogen atom, an -NH₂ group or a group -NHCOR7,
**R4** represents
a phenyl group,
a heteroaryl group chosen from an imidazolyl group optionally substituted with a (C1-C4)alkyl group, or a pyrazolyl group optionally substituted, on a carbon atom, with a phenyl group which may itself be substituted with one to three groups chosen from halogen atoms and (C1-C4)alkyl groups, or
a group -NR7R8, in which **R7** and **R8** represent, independently of each other, a (C1-C4)alkyl or (C1-C4)alkoxy group, or together form a saturated 5- to 7-membered ring, this ring possibly being substituted with a (C1-C4)alkyl group on a carbon atom or on a nitrogen atom, including on the nitrogen atom to which the groups R7 and R8 are attached to form a quaternary ammonium, such as a 1-piperidyl group,
in the form of enantiomers, diastereoisomers or mixtures of these various forms, including racemic mixtures, and also in the form of bases or addition salts with pharmaceutically acceptable acids.

4. Compounds of formula (I) according to Claim 1, **characterized in that R1, R2, R2', R9, R9'** and **m** are as defined in Claim 1 and **in that**:
**when X represents a nitrogen atom:**
**R3** is as defined in Claim 1, and
**R4** represents:
a 4-piperidyl group substituted, on a carbon atom or on the nitrogen atom, with a (C1-C4)alkyl group,
a group -(CH₂)ₚ-NR5R6 or -(CH₂)ₚ-CONR5R6, in which p may range from 1 to 4 and in which **R5** and **R6** represent, independently of each other, a (C1-C4)alkyl group,
a group -CO-(CH₂)ₚ-NR5R6, in which p may range from 0 to 4 and in which **R5** and **R6** represent, independently of each other, a hydrogen atom or a (C1-C4)alkyl group;
a group -(CH₂)ₚ-phenyl, in which p may range from 1 to 4 and in which the phenyl nucleus is optionally substituted with one to three groups chosen, independently of each other, from: a (C1-C4)alkyl group, a nitro group, a hydroxyl group, an amino group, a halogen atom, a trifluoromethyl group, a (C1-C4)alkoxy group, a (C1-C4)alkylamino group, a (C1-C4)dialkylamino group, an -NHCHO group or a group - NHCOR, in which **R** represents a (C1-C4)alkoxy group or a (C1-C4)alkyl group, this (C1-C4)alkyl group possibly being substituted with a dimethylamino group,
a group - (CH₂)ₚ-morpholinyl, - (CH₂)ₚ-pyrrolidinyl or -(CH₂)ₚ-tetrahydroisoquinoline, in which p may range from 1 to 4,
a group -(CH₂)ₚ-heteroaryl, in which p may range from 1 to 4 and in which the heteroaryl group is chosen from pyridyl, aminopyridyl, pyrimidinyl, pyrazinyl, pyridazinyl, imidazolyl and pyrazolyl groups, the said heteroaryl group possibly being substituted, on a carbon atom and/or on a nitrogen atom, with one to three groups chosen from a (C1-C4)alkyl group or a phenyl group,
a heteroarylcarbonyl group, the heteroaryl group being chosen from furyl, pyridyl, pyrimidyl, pyrazinyl, pyridazinyl and imidazolyl groups,
a phenylcarbonyl group, the phenyl group possibly being substituted with a halogen atom,
a (C1-C6)alkylcarbonyl group,
a group -(CH₂)ₚ-COOR' in which p may range from 0 to 4 and in which **R'** represents a hydrogen atom or a (C1-C6)alkyl group,
a phenylsulphonyl group optionally substituted on the phenyl nucleus with a halogen atom, a trifluoromethyl group, a (C1-C4)alkyl group, a nitro group or a (C1-C4)alkoxy group, or
a group -CO-(CH₂)ₚ-NR5R6, in which p may range from 0 to 4 and in which R5 and R6 represent, independently of each other, a hydrogen atom or a (C1-C4)alkyl group;
**and when X represents a carbon atom:**
• either **R3** represents a group -NR5R6, in which **R5** and **R6** represent, independently of each other, a (C1-C4)alkyl group; a group -NHCOR7, in which **R7** represents a (C1-C4)alkoxy group; a group -CONHR5, in which **R5** represents a hydrogen atom or a (C1-C4)alkyl group; a group -COR7, in which **R7** represents a (C1-C4)alkyl or (C1-C4)alkoxy group; or an -NHCONH₂ group, **R4** being as defined above in Claim 1,
• or **R3** is as defined above in Claim 1 and **R4** represents:
a phenyl group substituted with one to three groups chosen, independently of each other, from: a (C1-C4)alkyl group or a trifluoromethyl group,
an imidazolyl group substituted with a (C1-C4)alkyl group,
a pyrazolyl group substituted, on a nitrogen atom or on a carbon atom, with a phenyl group which may itself be substituted with one to three groups chosen from halogen atoms and (C1-C4)alkyl groups,
a group -CH₂-NR7R8, in which **R7** and **R8** are as defined in Claim 1, or
a group -NR7R8, in which **R7** and **R8** represent, independently of each other, a (C1-C4)alkyl group or together form a saturated 5- to 7-membered ring optionally comprising an additional nitrogen atom, this ring possibly being substituted with a (C1-C4)alkyl group on a carbon atom or on a nitrogen atom, including on the nitrogen atom to which the groups R7 and R8 are attached to form a quaternary ammonium, among which mention may be made of a 1-piperidyl group, a 1-pyrrolidinyl group or a 1-piperazinyl group,
in the form of enantiomers, diastereoisomers or mixtures of these various forms, including racemic mixtures, and also in the form of bases or addition salts with pharmaceutically acceptable acids.

5. Process for preparing the compounds of formula (I) according to any one of Claims 1 to 4, **characterized in that** a derivative of formula (II) 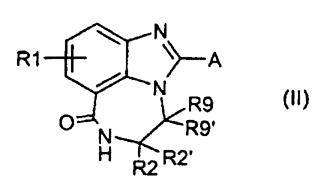 in which R1, R2, R2', R9 and R9' are as defined in Claim 1 and A represents a leaving group, is reacted in the presence of an amine of formula (III) 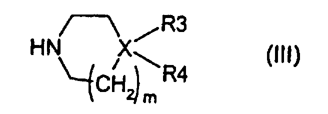 in which X, R3, R4 and m are as defined in Claim 1, in a solvent which may be an alcohol, an ether, dimethylformamide or a hydrocarbon, to obtain the compound of formula (I) according to Claim 1, the reaction possibly taking place in the presence of a base, in the presence of alkali metal halides or in the presence of palladium-based or nickel-based catalysts.

6. Compounds of formula (II) as defined in Claim 5, which are useful as synthetic intermediates.

7. Medicinal product, **characterized in that** it comprises a compound of formula (I) according to any one of Claims 1 to 4.

8. Pharmaceutical composition, **characterized in that** it comprises a compound of formula (I) according to any one of Claims 1 to 4, or a pharmaceutically acceptable salt thereof, and also one or more suitable pharmaceutical excipients.

9. Use of a compound of formula (I) according to any one of Claims 1 to 4, to prepare a medicinal product for treating or preventing disorders in which the enzyme poly(ADP-ribose)polymerase is involved.
